# EUROPEAN PATENT APPLICATION

(11) **EP 2 730 644 A2**
(43) Date of publication of application: **14.05.2014**
(21) Application number: 14152939.6
(22) Date of filing: 04.06.2009
(51) Int. Cl.: C12M 3/06

(54) **Organ-on-a-chip-device**

(30) Priority: 04.06.2008 US 58766 P
(62) Divisional of application: 09757294.5
(71) Applicant: TissUse GmbH, 15528 Spreenhagen (DE)
(72) Inventor: Marx, Uwe, 15528 Spreenhagen (Markgrafpieske) (DE)
(74) Representative: Zwicker, Jörk

(57) **Abstract**

The present invention relates to a self-contained sensor controlled organ on a chip-device, which allows establishing or maintaining organs or organoids as well as stem cell niches in a miniaturized chip format, suitable for online observation by live cell imaging and for example two photon microscopy and their use for, e.g. testing the activity, pharmacodynamic and pharmacokinetic of compounds or to study self-assembly, homeostasis, damage, regeneration or interaction of organs or organoids and stem cell niches, as well as phenomena of maturation, aging, death and chronobiology.

## Description

The present invention relates to a self-contained, preferably sensor controlled organ on a chip-device, which allows establishing or maintaining organs or organoids as well as stem cell niches in a miniaturized chip format, suitable for online observation by live cell imaging and for example two photon microscopy and their use for, e.g. testing the activity, pharmacodynamic and pharmacokinetic of compounds or to study self-assembly, homeostasis, damage, regeneration or interaction of organs or organoids and stem cell niches, as well as phenomena of maturation, aging, death and chronobiology.

### Prior Art

A paradigm of stringent correlation between architecture and functionality applies to all levels of biological existence on earth. These levels of increasing biological complexity appeared step by step within a multi-million year process of evolution. Existence was most likely triggered by slight changes of external environment which created the ability for self-assembly to the next level of complexity. For humans, molecules, cells, organoid tissues, organs, systems and finally the individual organisms themselves were thought to represent these levels. Nowadays, it has been proven that almost all organs and systems are built up by multiple, identical, functionally self-reliant, structural units. These organoid units are of very small dimensions, from several cell layers up to a few millimetres. Liver lobuli, nephrons of kidney, dermis and epidermis of skin, gut mucosa, Langerhans islets of pancreas, grey and white matter of brain cortex and cerebellum and adult quiescence-promoting stem cell niches are a small selection of examples of such human organoid structures, all with a prominent functionality and highly variable conglomerate geometry. Due to distinguished functionality, a high degree of self-reliance and multiplicity of such micro-organoids within the respective organ, their reactivity pattern to any substances seems to be representative of the whole organ. Nature created very small but sophisticated biological structures to realize most prominent functions of organs and systems. Multiplication of these structures within a given organ is nature's risk management tool to prevent total loss of functionality during partial organ damages. On the other hand, evolutionarily this concept has allowed the easy adjustment of organ size and shape to the needs of a given species - for example liver in mice and man - still using nearly the same master plan to build up the single functional micro-organoid unit. A unique and outstanding chance for substance testing predictive to human exposure lies in the establishment of equivalents of human micro-organoids in vitro. A first organ on a chip device, called Integrated Discrete Multiple Organ Cell Culture, was described 2004 by Li et al Chem. Biol. Interaction. This device is based on static cultures of different tissues in a conventional 6 well plate covered with a gel, connecting different cultures through a diffusion based semisolid medium. Since that time significant efforts were made to develop culture systems and bioreactors, more naturally emulating architecture and *in vivo* environment *in vitro.* A comprehensive summary is given by M.A. Swartz et al: Capturing complex 3D tissue physiology in vitro. Nat. Rev. Mol. Cell Biol., 7, 211-224, 2006. Miniaturized perfused culture systems were developed for a number of different tissues, e.g. for renal tubuli (Minuth et al: The formation of pores in the basal lamina of regenerated renal tubules. Biomaterials, 29, 2749-2756, 2008) or for neuronal tissue (Hillenkamp et al: Maintenance of adult porcine retina and retinal pigment epithelium in perfusion culture: Characterization of an organotypic in vitro model. Experimental Eye Research, 86, 661-668, 2008).

None of the existing 3D culture systems and bioreactors were designed to meet the requirements regarding size, shape and nutrition requirements of different organoids in a self-containing and online observable chip environment, independent of external equipment. By applying the present invention for example to the creation of human organoids, a new quality of biosafety and efficacy testing for substances, such as chemicals, drugs, nutraceuticals and cosmeceuticals can be envisioned prior to exposure in man.

### Summary of the Invention

The present invention relates to a self-contained organ-on-a-chip device (1) comprising:
(a) at least one medium feed reservoir (2),
(b) at least one organ growth section (3) comprising at least one organ cavity (4, 4a, 4b), and
wherein the medium feed reservoir (2) is connected to the at least one organ growth section (3) by a microfluidic feed channel (6).

In a further aspect the present invention relates to a self-contained organ-on-a-chip device (1) comprising
(a) at least one organ growth section (3) comprising at least one organ cavity (4, 4a, 4b), and
(b) wherein the at least one organ cavity (4, 4a, 4b) comprises and/or is connected to at least one sensor (8, 8a, 8b).

In a further aspect the present invention relates to a self-contained organ-on-a-chip device (1) comprising:
(a) at least one organ growth section (3) comprising at least one organ cavity (4, 4a, 4b), and
(b) wherein the organ growth section (3) comprises at least one stem cell cavity (9).

In a further aspect the present invention relates to a method of manufacturing a self-contained organ-on-a-chip device (1) of the present invention, comprising the steps of bonding a medium layer (12) fluid-tight to a growth section layer (13) or parts thereof.

In a further aspect the present invention relates to a supply unit (17) for holding the self-contained organ-on-a-chip device (1) of the present invention during operation comprising: (a) holding means (18) for releasably engaging the self-contained organ-on-a-chip device (1), and (b) electric connectors (19) for connecting to corresponding connectors on the self-contained organ-on-a-chip device (1) to the supply unit (17).

A method of establishing an organ and/or organoid in a self-contained organ-on-a-chip device (1) of the present invention, comprising the steps of:
(a) loading a suspension of cells and/or a tissue slice into one or more organ cavities (4, 4a, 4b) and
(b) fluid-tight sealing of the one or more organ cavities (4, 4a, 4b).

In a further aspect the present invention relates to method of testing the effect of one or more test compounds on one or more tissues, organs and/or organoids established in a self-contained organ-on-a-chip device (1) of the present invention, comprising:
(a) providing a self-contained organ-on-a-chip device (1) of the present invention comprising one or more tissues, organs and/or organoids or
   carrying out the method of establishing a organ and/or organoid in a self-contained organ-on-a-chip device (1) of the present invention,
(b) adding one or more test compounds to the organ and/or organoid
(c) assessing the organ and/or organoid microscopically and/or
   determining one or more parameter determinable by one or more sensors (8, 8a, 8b).

In a further aspect the present invention relates to the use of the self-contained organ-on-a-chip device (1) of the present invention comprising one or more tissues, organs and/or organoids for testing the effects of one or more test compounds on the tissues, organs or organoids or for examining organ or organoid functions.

### Brief Description of the Figures

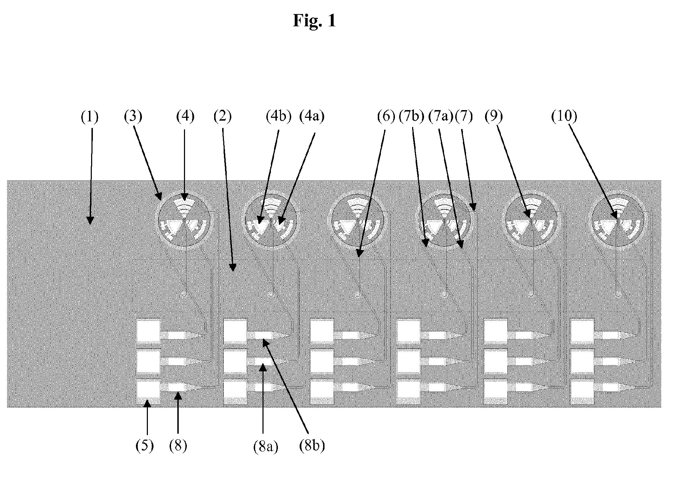

- Fig. 1:: Top-down view of a preferred embodiment of a section of a partly assembled self-contained organ-on-a-chip device (1) comprising the upper closing layer (14) and the organ cavity layer (15). Since the upper closing layer (14) and the organ cavity layer (15) are on top of each other they can not be distinguished in the top-down view depicted here and, accordingly the upper closing layer (14) and the organ cavity layer (15) are not labelled in this figure. This section comprises six individual organ growth sections (3), each comprising three organ cavities (4, 4a, 4b). To reveal the features comprised therein the parts are drawn translucent. However, in some preferred embodiments the material used to produce the upper closing layer (14) and/or the organ cavity layer (15) is partially or entirely translucent. The medium fed from the upper medium layer (12) (not shown) flows through the microfluidic feed channel (6), preferably to the centre of an organ growth section (3) to allow even distribution of the medium to the one, two, three or more organ cavities (4, 4a, 4b) comprised in one organ growth section (3). Preferably the medium is fed into the organ growth section from an outlet (10) positioned opposite to the stem cell cavity (9), which is located in the organ cavity layer (15). Thus, stem cells may flow with the fresh medium into the adjacent organ cavities (4, 4a, 4b) to replenish/regenerate the cell populations constituting the respective organ and/or organoid. The organ cavities (4, 4a, 4b) of one organ growth section (3) are preferably populated by different cell populations forming different tissues, organs and/or organoids, which allows, e.g. the testing of the effect of one compound on more than one organ or organoid simultaneously. The organ cavities (4, 4a, 4b) are preferably microstructured to support the organization of the cell population into the respectively desired organ and/or organoid. Some tissues, organs and/or organoids will require a particular microenvironment, e.g. changing pressure, secondary flow of medium within the organ cavity, special additional medium etc., to form and/or to be maintained. Organ cavity (4) is structured to provide several separate microcavities, which supports the establishment and/or maintenance of, e.g. neurons. Organ cavity (4a) is structured to provide a pressurized environment, which supports the establishment and/or maintenance of, e.g. bone and/or cartilage structures. Organ cavity (4b) is structured to provide a secondary flow within the organ cavity, which supports the establishment and/or maintenance of, e.g. vascularised skin. The organ cavity (4, 4a, 4b) is preferably delimited at the upper end by the upper closing layer (14) and at the lower end by the lower closing layer (16), while the sides of the cavity are formed in the organ cavity layer (15). Thus, microstructures required for organ growth and/or maintenance may also be provided by the upper and/or lower end of the organ cavity (4, 4a, 4b). Preferably the outlet allowing medium to flow into the microfluidic waste channel (7, 7a, 7b) is located at a position opposite to the outlet (10) of the microfluidic feed channel (6) in a way that any medium flowing from inlet (10) into the organ cavity (4, 4a, 4b) flows preferentially through the entire organ cavity (4, 4a, 4b) before it flows out of the organ cavity through the inlet of the waste channels (7, 7a,b). The waste medium then flows, preferably through a separate channel (7, 7a, 7b) for each organ cavity (4, 4a, 4b) within an organ growth section (3) to one or more sensors located in the flow path (8, 8a, 8b). Thus, the response to a given compound and/or environmental change can be assayed for each organ and/or organoid comprised in an organ cavity (4, 4a, 4b) of an organ growth section (3) individually. Thereafter the medium flows into the medium waste reservoir (5). While it is possible that a common medium waste reservoir (5) is provided for the waste medium of all organ cavities (4, 4a, 4b) of an organ growth section (3) or even for all organ growth sections of one self contained organ-on-a-chip device (1), it is preferred that one medium waste reservoir (5) is provided for the waste medium of each organ growth section or preferably for each organ cavity (4, 4a, 4b), to avoid mixing of the waste medium. It is also preferred that all organ cavities having the same microstructures within one organ growth sections (3) or within different organ growth sections (3) are connected to one waste medium to avoid mixing of waste from different organs or organoids. In a preferred embodiment, wherein each organ cavity (4, 4a, 4b) of the organ growth sections (3) is connected to a separate waste reservoir (5) it is possible to withdraw a sample or all of the waste medium from the individual waste medium reservoirs (5) and further analyze each waste medium from one organ and/or organoid individually. The waste medium reservoir (5) cavity is preferably located in the medium layer (12), which is not shown. In the preferred embodiment depicted in this figure a corresponding rectangular opening is provided in the upper closing layer (14) and the organ cavity layer (15). Therefore, in this embodiment the medium waste reservoir (5) extends almost through the entire self contained organ-on-a-chip device (1) between the bottom of the lower closing layer (16) and the upper end of the medium layer (12), thus, providing maximal space for holding the waste medium.

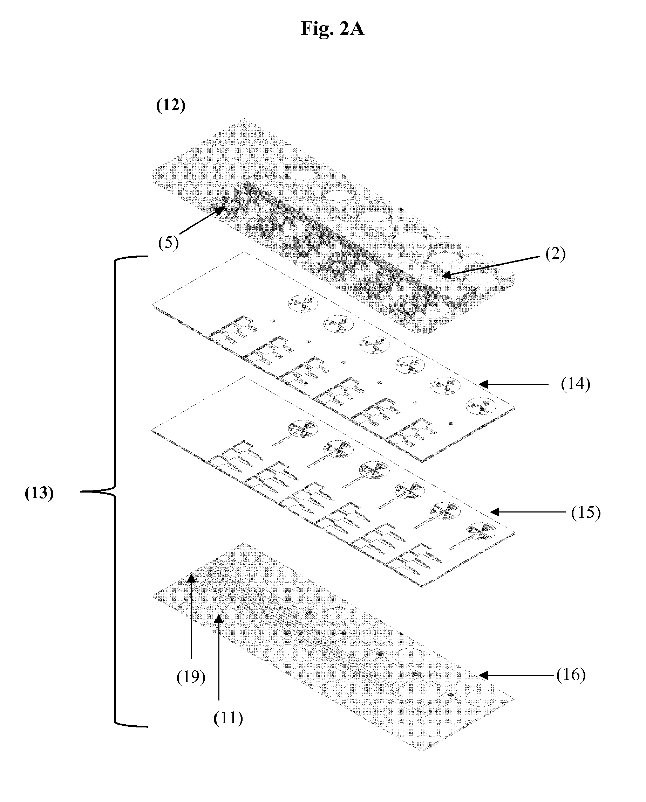

- Fig. 2A:: Exploded view of a preferred embodiment of a self-contained organ-on-a-chip device (1) comprising the medium layer (12), the organ growth section layer (13) comprising an upper closing layer (14), the organ cavity layer (15) and the lower closing layer (16). The medium layer (12) comprises cut outs to allow access to the organ growth sections (3), located in the organ growth section layer (15) and between the upper and lower closing layer. These cut-outs are preferably commensurate in size with the size of the cut-outs of the respective organ growth section (3) located beneath to allow access to each organ cavity (4, 4a, 4b) within an organ growth section (3). Preferably the cell population, preferably a cell suspension and/or tissue slice, used to establish the respective organ or organoid is directly loaded into the organ cavity (4, 4a, 4b) through this cut-out, which is sealed thereafter, to avoid contamination of the cell populations loaded. Preferably this seal is fluid tight but gaspermeable. Alternatively, the entire cell population is generated from one or more stem cells, which may be introduced into the organ growth section through the microfluidic feed channel (6) together with the medium and/or through an additional access port directly into the stem cell cavity (9). Furthermore a medium feed reservoir (2) is located within the medium layer (12). This reservoir is preferably provided with an access port to allow the supply of the required media into the medium feed reservoir (2) or the medium layer (12) may be provided with a prefilled medium feed reservoir, which may be provided with an opening to allow air to enter the medium feed reservoir. For flexibility the medium layer (12) comprising a prefilled medium feed reservoir (2) may be connected to the organ growth section layer (13) t at the point of use to form the self-contained organ-on-a-chip device (1) or the completely assembled self-contained organ-on-a-chip device may be provided either with a prefilled medium feed reservoir (2) or with an empty medium feed reservoir (2) that is filled at the point of use. Furthermore the medium layer (12) comprises one or more medium waste reservoirs (5). These are in fluidic connection to the organ growth section (3) and in particular to the organ cavities (4, 4a, 4b) comprised therein. It is preferred that a sensor (8, 8a, 8b) is located in the flow path (7, 7a, 7b) connecting the individual organ cavities with the medium waste reservoir(s) (5), preferably located within the self-contained organ-on-a-chip device (1). In the preferred embodiment depicted in this figure a cut-out of similar shape and size is provided in the upper closing layer, the organ cavity layer and the medium layer to form the medium waste reservoir (5). The lower closing layer (16) is provided with electric connectors (19) to provide (i) power to heating means (11), which may be located at the bottom of the organ cavities (4, 4a, 4b), the medium feed reservoir (2) or which may be positioned in any other part of the lower closing layer; and/or (ii) to connect to sensor devices and/or actuators (pressurizing means, pumps, temperature sensors etc.), which are preferably located within the organ cavities (4, 4a, 4b) or which may be positioned in any other part of the lower closing layer; and/or (iii) to connect to sensors (8, 8a, 8b).

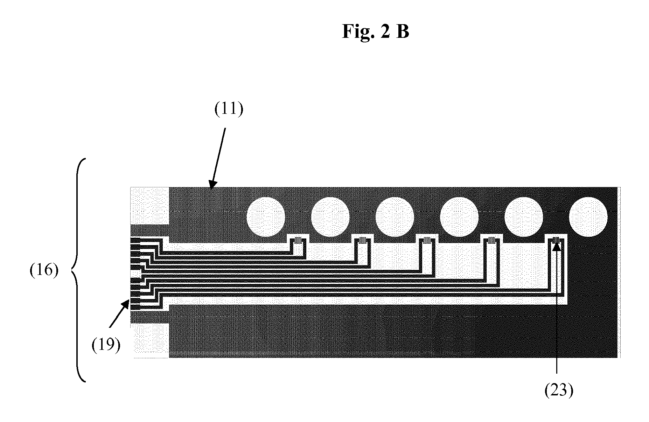

- Fig. 2B: Top-down view of the upper side of the lower closing layer (16). Depicted are heating means (11), which are preferably made of indium tin oxide (ITO), a temperature sensor (23), which is preferably a meander structure made of platinum, and electric connectors (19), which are preferably made of gold. Similarly the conductive paths are made of gold. The lower closing layer (16) is preferably made of glass and translucent at least in the regions of the organ growth sections (3) to allow transmission microscopy. Preferably the lower closing layer (16) is provided with temperature sensors to control the temperature within the organ growth sections (3).

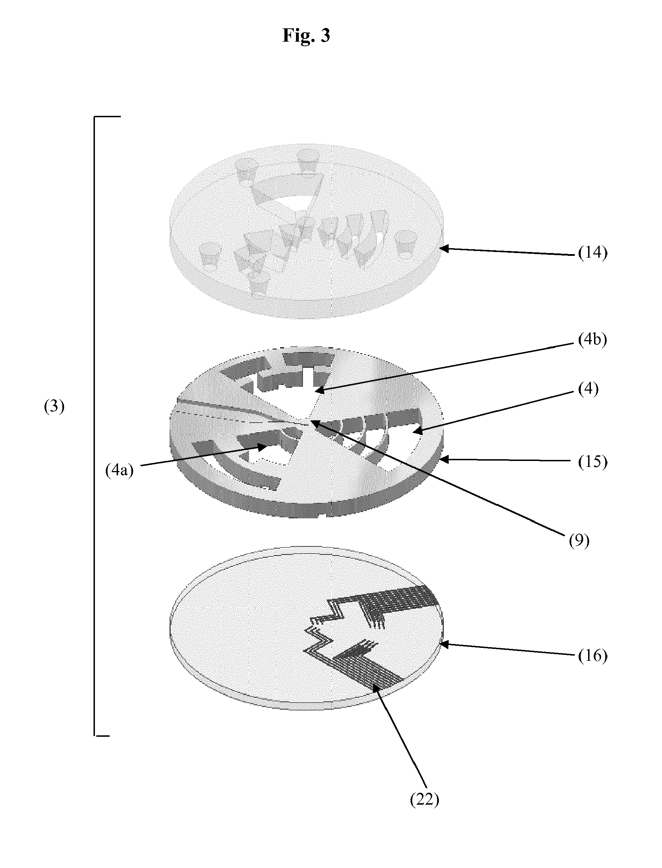

- Fig. 3: Exploded view of a preferred embodiment of an organ growth section (3) comprising three organ cavities (4, 4a, 4b). In this preferred embodiment the organ cavities (4, 4a, 4b) are each closed or at least partially closed on the upper side by the upper closing layer (14), which comprises microstructures, the organ cavity layer (15) that provides the majority of the microstructures required and the lower closing layer (16), which provides, e.g. impedance measuring means (22) to assess the impedance in an organ cavity adapted to nerve growth.

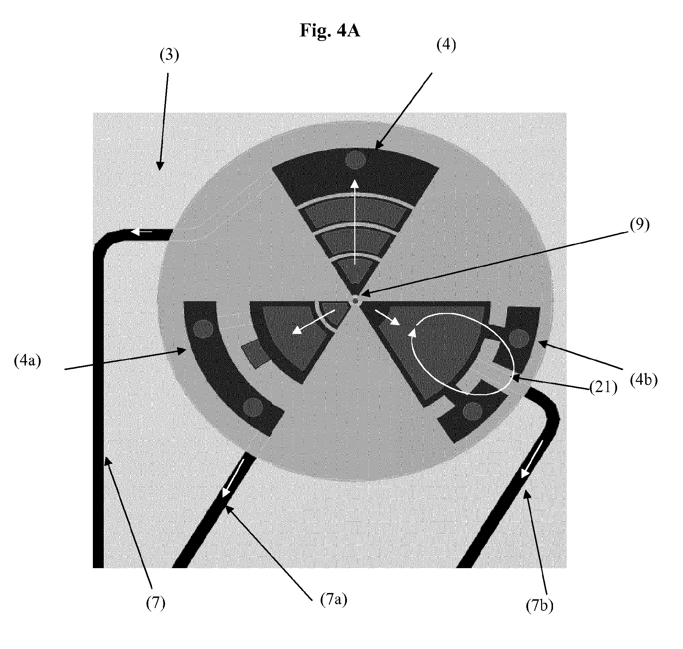

- Fig. 4A: Top-down view on a section of a preferred embodiment of the organ cavity layer (15) comprising an organ growth section (3) comprising three differently structured organ cavities (4, 4a, 4b). The medium flow within the organ growth section (3) into the organ cavities (4, 4a, 4b) starts from the outlet (10) of the microfluidic feed channel (not shown, since it is located on the upper closing layer in this embodiment), which is juxtaposed to the stem cell cavity (9), into the organ cavities (4, 4a, 4b) and out through three separate microfluidic waste channels (7, 7a, 7b). The direction of the fluid flow is depicted by straight white arrows. Preferably the flow within the organ cavities is radially outwards from the medium outlet in the middle of the growth section towards the inlets of the waste channels (7, 7a, 7b) at the periphery of the growth section. In growth cavity (4b), which provides an environment for establishment/maintenance of vascularised skin a secondary fluid flow (21) is effected by pressurizing means or pumps located in the side chambers of organ cavity (4b).

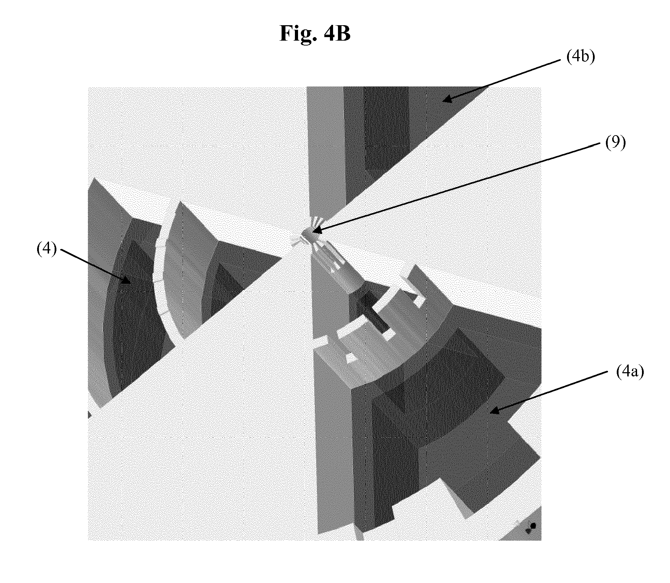

- Fig 4B: Three dimensional view of part of an organ growth section (3) comprising three organ cavities (4, 4a, 4b), wherein a preferred embodiment of an adult stem cell cavity (9) is positioned in the center of three organ cavities (4, 4a, 4b).

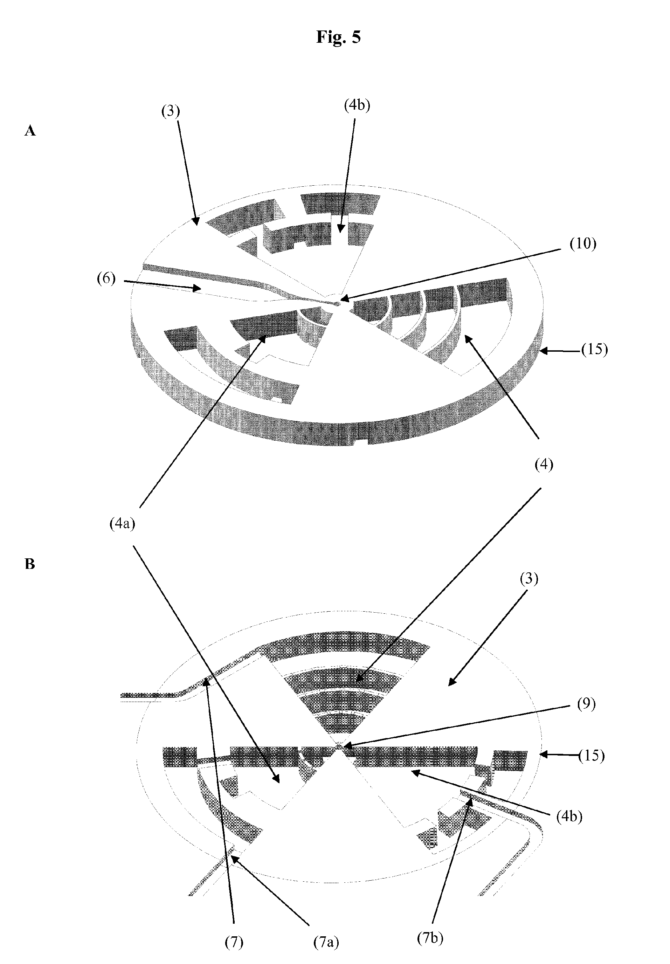

- Fig. 5: Top (A) and Bottom (B) view of a section of a preferred organ cavity layer (15) comprising the middle segment of an organ growth section (3) comprising three organ cavities (4, 4a, 4b). The upper and lower sealing of the organ cavities (4, 4a, 4b) is provided by the upper and lower closing layer, respectively, which are not shown. Panel A depicts the microfluidic feed channel (6) which ends in the outlet (10). Panel B depicts the stem cell cavity (9), which is located opposite to the outlet (10).

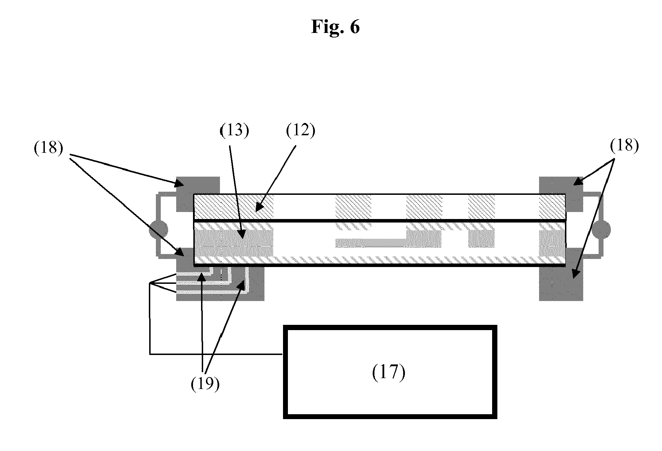

- Fig. 6: Sectional view of a preferred embodiment of self-contained organ-on-a-chip device (1). Depicted is the medium layer (12) and organ growth section layer (13), which are held in place by holding means (18), which also provide at least one contact surface comprising electric connectors (19) that releasably connect to corresponding connectors on the bottom side of the self-contained organ-on-a-chip device (1). A supply unit (17) provides power for, e.g. heating, pumping and/or electric stimulation and preferably comprises a data processing unit to evaluate and/or indicate signals from one or more sensors.

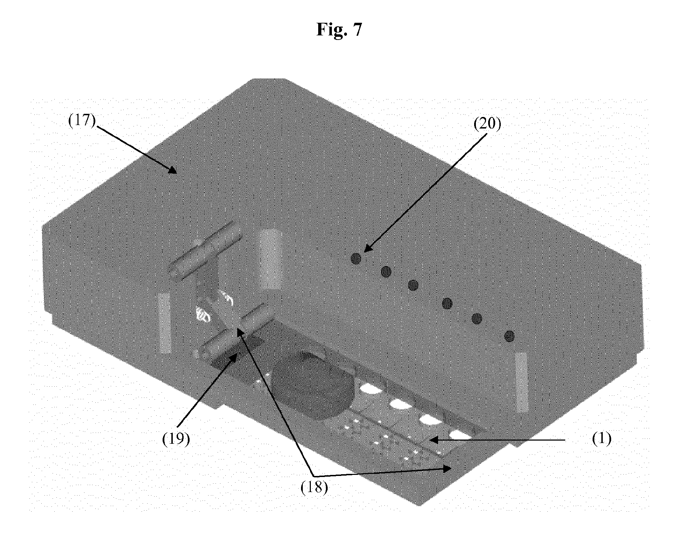

- Fig. 7: Three-dimensional view of a preferred embodiment of an integrated supply unit (17) comprising holding means (18) on both sides of the self-contained organ-on-a-chip device (1). Electric connectors (19) connecting the self-contained organ-on-a-chip device (1) to the supply unit (17) and overheat indicator means (20) indicating excess heat in the respective organ growth sections (3).

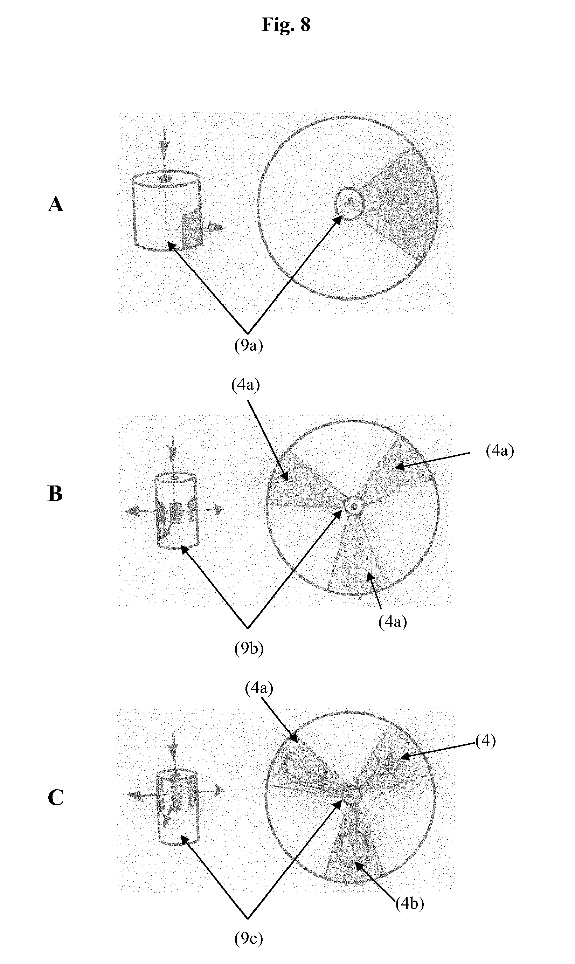

- Fig. 8: The three Panels A, B and C of Fig. 8 each show a three-dimensional view and a cross-section of different stem cell cavities (9). Panel A shows an exemplary neonatal stem cell niche cavity (9a); Panel B shows an exemplary pre/postnatal stem cell niche cavity (9b); and Panel C shows an exemplary adult quiescence-promoting stem cell niche cavity (9c).

### Detailed Description of the Invention

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended items. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. In the following, some definitions of terms frequently used in this specification are provided. These terms will, in each instance of its use, in the remainder of the specification have the respectively defined meaning and preferred meanings:
"Autocrine factors": are all those substances secreted by cells, which support and mediate maintenance, growth or differentiation of the same cell that secreted the factor.
"Paracrine factors": are all those substances secreted by a cell, which support and mediate maintenance, growth and differentiation of another but adjacent cell.
"Self-conditioning" describes all factors leading to improved cell behaviour.
"Differentiation" means the development of tissue specific functions of cultured cells.
"Maintenance" describes the ability to keep all functions of a given tissue constant within a given cell culture process.
"Living cell material" describes cells, cell aggregates, tissues, organoids and organs.
"Cells" means cell lines or primary cells of vertebrates or invertebrates.
"Tissue" stands for biopsy material or explants taken from patients or animals.
"Organoids" means artificial, *de novo* generated, functional cell aggregates of different types of cells *in vitro* that show at least one organ or tissue function, preferably shows the majority of organ or tissue functions.
"Organ" means artificial, *de novo* generated, functional cell aggregates of different types of cells *in vitro* that show all functions of the natural organ.
"Medium" (plural form: "media") means growth supporting liquid with nutrients and substances for cultivation of cells.
"Supplements" describe substances to be added to culture media in order to induce or modify cell function, which may have a defined composition like, e.g. purified or recombinant cytokines or growth factors, or which are undefined like, e.g. serum.
"Matrix" means substances or mixtures of substances, which enhance proliferation, differentiation, function or organoid or organ formation of cells. Matrix material may be coated on surfaces or may be provided in voluminous applications to optimize cell attachment or allow three-dimensional cultures. Matrix usable in the context of the present invention can take a variety of shapes comprising, e.g. hydrogels, foams, fabrics or non-woven fabrics. The matrix material may comprise naturally occurring matrix substances like extracellular matrix proteins, preferably collagens, laminins, elastin, vitronectin, fibronectin, small matricellular proteins, small integrin-binding glycoproteins, growth factors or proteoglycans or may include artificial matrix substances like non degradable polymers such as polyamid fibres, methylcellulose, agarose or alginate geles or degradable polymers, e.g. polylactid.
"Microfluidics" relates to the behaviour, precise control and manipulation of fluids that are geometrically constrained to a small, typically sub-millimetre, scale. Microfluidics means one or both of (i) small volumes (µl, nl, pl, or fl), i.e. the organ cavities preferably have a volume of 1 mm³ or less and the microfluidic channels are capable of allowing the flow of between 0.1 to 2 mm³ medium per day at a pressure of 0.005 to 2 Bar, i.e. 0.05, 0.1, 0.2, 0.3, 0.4, 0.5, 1.0, 1.5 or 2.0 Bar and (ii) small size, i.e. channel diameter of around 100 nanometers to several hundred micrometers. In the context of the present invention a microfluidic channel preferably has a diameter of between 100 nm to 1 mm, preferably between 0.5 µm to 200 µm, more preferably between 1 µm to 100 µm, i.e. 1, 2, 3, 4, 5, 6, 7, 8, 9, 10, 15, 20, 25, 30, 35, 40, 45, 50, 55, 60, 65, 70, 75, 80, 85, 90, 95 or 100 µm. If the opening of the channel does not have a circular cross-section then the opening preferably has a surface area that is within the ranges and preferred ranges of surface areas for channels with circular cross sections as indicated above.

To overcome the problems associated with prior art cell culturing systems, the present invention provides a self-contained organ-on-a-chip device (1) comprising:
(a) at least one medium feed reservoir (2),
(b) at least one organ growth section (3) comprising at least one organ cavity (4, 4a, 4b),
   and
wherein the medium feed reservoir (2) is connected to the at least one organ growth section (3) by a microfluidic feed channel (6).

The term "self-contained" refers to the fact that media and supplements required for differentiation and maintenance of organs, tissues or organoids in the at least one organ growth section (3) are provided from within the organ-on-a-chip device (1), i.e. at least one medium reservoir (2) is comprised within the organ-on-a-chip device (1) and is connected through microfluidic channels (6) within the organ-on-a-chip device (1) to the organ growth section (3) and/or to the one or more organ cavities (4, 4a, 4b) comprised within the one or more organ growth sections (3). Thus, there is no fluidic connection providing fluid from an external fluid reservoir. Accordingly, the self-contained organ-on-a-chip device (1) can be handled and moved, without the danger of contaminating the medium and subsequently the cells within the organ growth sections (3). Additionally, it is preferred that gaseous medium, e.g. O₂/CO₂, is provided to the organ growth section in a passive manner, i.e. by diffusion into the medium through a membrane or biocompatible polymer foil from the environment. This membrane or polymer foil is preferably fluid-tight. Again this is preferred to allow the handling of the organ-on-a-chip device. Preferably the membrane or foil covers at least partially the organ growth section (3), thus allowing O₂/CO₂ to diffuse into the medium flowing through the organ cavities. In a preferred embodiment the membrane is formed or attached after cells have been loaded into the organ cavities or it forms an integral part of the organ-on-a-chip device. Accordingly, in a preferred embodiment the organ-on-a-chip device comprises no connectors to an external gaseous medium supply and/or does not comprise a device for actively aerating the medium. Preferably, the medium is not recirculated through the organ growth section (3) but is flown from one or more medium reservoirs (2) through the organ growth sections (3) into one or more medium waste reservoirs (5).

An "organ-on-a-chip device" refers to an assembly, which is preferably made from multiple individually structured and microstructured layers, that are in fluid-tight connection with each other and is preferably capable to provide a fluid-tight environment and, thus, preferably sterile environment. The device is preferably dimensioned to be used in standard high throughput set ups, e.g. having the size of a standard microtiterplate or strip. Thus, preferably the width is between 2 to 10 cm, preferably 1, 2, 3, 4, 5, 6, 7, 8, 9, or 10 cm and/or the length between 3 and 15 cm, preferably 3, 4, 5, 6, 7, 8, 9, 10, 11, 1, 12, 13, 14 or 15 cm and/or the height between 0.2 and 10 mm, more preferably between 1 and 4 mm, i.e. 0.2, 0.3, 0.4, 0.5, 0.6, 0.7, 0.8, 0.9, 1.0, 1.1, 1.2, 1.3, 1.4, 1.5, 1.6, 1.7, 1,8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 5.0, 6.0, 7.0, 8.0, 9.0 or 10.0 mm. To conform to the standard mictrotiterplate format the width to length are preferably in a ratio of about 1:3. Particularly preferred is a size of 2.5 cm width, 7.5 cm length and 3 mm height.

Preferred materials comprise SiO₂, glass, and synthetic polymers. Preferred synthetic polymers comprise polystyrol (PS), polycarbonate (PC), polyamide (PA), polyimide (PI), polyetheretherketone (PEEK), polyphenylenesulfide (PPSE), epoxide resin (EP), unsaturated polyester (UP), phenol resin (PF), polysiloxane, e.g. polydimethylsiloxane (PDMS), melamine resin (MF), cyanate ester (CA), polytetrafluoroethylene (PTFE) and mixtures thereof. Particularly preferred synthetic polymers are optically transparent and include, e.g. polystyrol (PS), polycarbonate (PC), and polysiloxane, e.g. polydimethylsiloxane (PDMS).

An organ growth section (3) is a microstructured region within the organ-on-a-chip device (1) that provides the entire micro-environment for organoid and/or organ differentiation and/or maintenance, including preferably medium inlet, medium outlet, stem cell cavity (see below), sensors (see below), an organ cavity (4) (see below) that holds the majority of the cells forming the respective organoid or organ, and/or an open surface, which may be covered in an essentially fluid-tight and gaspermeable or fluid-tight and gaspermeable way by appropriate means, including a membrane, e.g. PTFE membranes, fibrin sheets, spray-on band aid sheets and or sheets of coagulation products, once the cells/tissues have been loaded into the organ growth section (3) or by flexible sheets that cover the opening, e.g. lips made from flexible material like polysiloxane, e.g. PDMS. In a preferred embodiment such flexible sheet will cover the entire organ growth section and will have cuts in the areas of each organ cavity (4, 4a, 4b) allowing access through the cut to the individual organ cavity (4, 4a, 4b). The flexible sheets have the advantage that the organ growth sections (3) remain accessible without the necessity to reseal the membrane after access. Preferably the covered surface is fluid-tight but gaspermeable and, thus, allows exchange of O₂ and CO₂ between the cells in the organ growth section and the environment. Preferably the organ growth section has an essentially circular or a circular form, which is advantageous when the organ growth section comprises more than one organ cavities. In this preferred embodiment the organ growth section has essentially the form of a flat cylinder, which however, is not entirely hollow but comprises the structures and microstructures outlined throughout this specification. The ration of diameter to height of an organ growth section is preferably between 2:1 1 to 6:1, more preferably between 3:1 1 to 5:1. In particular, if it comprises two, three, four, five, six, seven, eight or more organ cavities (4, 4a, 4b) the circular structure is advantageous since it is possible to provide medium through a microfluidic feed channel (6) that has an outlet (10) in the center of the circle. The medium will then be distributed evenly between the organ cavities (4, 4a, 4b), which each have the form of a segment of a circle and viewed three-dimensionally the form of a segment of a cylinder. Preferably, a growth section has a surface of 0.1. to 3 cm² preferably 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0 cm², particularly preferred growth sections have a surface area of between 0.3 to 0.7 cm², preferably 0.56 cm². If the growth section has a circular shape it is preferred that it has a diameter of between 0.1 and 1 cm, preferably 0.1, 0.2, 0.3, 0.4, 0.5, 0.6, 0.7, 0.8, 0.9, or 1.0, most preferably 0.6 cm. Typically an organ-on-a-chip device will comprise more than one organ growth section (3). Given the indicated preferred sizes of each organ growth section (3) it is possible to fit large numbers of separate organ growth sections on one organ-on-a-chip device. Preferably one organ-on-a-chip device comprises between 3 and 2000 organ growth sections (3), preferably 3, 4, 5, 6, 7, 8, 9, 10, 11, 12, 13, 14, 15, 16, 17, 18, 19, 20, 24, 30, 36, 48, 60, 72, 84, 96, 108, 120, 132, 144, 156, 168, 180, 192, 204, 216, 228, 240, or more. In the preferred microtiterplate-like format 6, 24, 96, 384 or even 1536 organ growth sections (3) are arranged in a 2:3 rectangular matrix on the organ-on-a-chip device.

As set out above an organ growth section (3) comprises a cavity termed "organ cavity" which holds the majority of the cells, i.e. at least 80%, preferably 85%, 90%, 95%, 98% or more of the cells comprised in the organ growth section. The organ cavity (4, 4a, 4b) preferably has the proper dimension, shape and nutrition for each specific organ and provides access to introduce additionally necessary elements of micro-architecture and micro-environment as well as to load the organ-on-a-chip device with the cell suspension, cell clusters and/or tissue slices, as the case may be, and is coated with the appropriate materials to attract/maintain cells of a particular type as outlined in more detail below. Additionally the organ cavity, which in fact may be subdivided to form several "sub-cavities", which may be required to simulate the correct environment for a particular tissue or organ type, may be equipped with sensors, microactuators etc. as explained in more detail below. It is preferred that each organ cavity within one growth section provides the appropriate microenvironment for a different organ and/or organoid, e.g. for neurons, heart tissue, cartilage, bone and/or vascularised skin. In this way it is possible to assess the effect of one particular compound on several tissues, organoids and/or organs simultaneously. Alternatively, one organ growth section can comprise two or more organ cavities of the same type, which will allow measuring the effect of a given compound with a higher statistical significance by averaging the results obtained from two, three, four or more organ cavities in parallel. In addition one organ growth section may comprise one organ cavity that comprises cells of a particular cell type, which may serve as a standard, for each measurement. Typically an organ cavity within an organ growth section has a volume between 1 x 10² to 0.01 mm³, preferably 100, 90, 80, 70, 60, 50, 40, 30, 20, 10, 9, 8, 7, 6, 5, 4, 3, 2, 1, 0.9, 0.8, 0.7, 0.6, 0.5, 0.4, 0.3, 0.2, 0.1, 0.09, 0.08, 0.07, 0.06 and 0.05 mm³, preferably 1 mm³. It is preferred that each organ cavity within a given growth section has a similar, e.g. ± 20%, or the same volume. If the growth section comprises two or more organ cavities (4, 4a, 4b), it is preferred that they are radially arranged with respect to the outlet (10) of the microfluidic feed channel (6). In this preferred embodiment the organ cavities (4, 4a, 4b) are preferably arranged in the form of conical segments of a disc, wherein the disc is the organ growth section (3).

The organ cavity preferably is substructured by subdivision into two, three, four or more cavities comprising or consisting of a main cavity and one or two side cavities, which are all in fluidic connection. It preferably comprises a structured internal surface providing ridges, channels, funnels, with the aim to delimit an environment suitable for supporting growth and maintenance of the respectively desired organoid, and/or organ. Thus, organ cavities (4, 4a, 4b) within an organ growth section (3) provide space for self-assembly, maintenance and/or re-assembly of the smallest functionally self-reliant structural unit of a specific organ (e.g. alveoli of lung, epidermis and dermis of skin, gut mucosa, liver lobulus, or nephron of kidney) or a specific system (e.g. microvasculature of blood system, grey matter of nerve system). Nature's principle building blocks for directed organ assembly *in vivo* are dimension, shape, nutrition characteristics, micro-architecture (e.g. extracellular matrices and membranes as well as surface properties) and local microenvironment (e.g. morphogen and chemokine gradients), which are simulated in the organ-on-a-chip device of the present invention. Preferably, the organ section comprises a combination of organ cavities with structures supporting growth of the following organ combinations: liver lobulus and epidermis and dermis of the skin, preferably comprising a hair follicle, optionally comprising a microvasculature connecting both organ cavities.

Preferred organ cavities (4, 4a, 4b) are designed to provide the appropriate environment for brain tissue (4), hybrid bone / cartilage organoid (4a) and vascularised skin (4b), preferably with hair follicle. Further preferred tissues are liver segments, kidney and mucosa of the intestines.

In a preferred embodiment of an organ cavity (4) designed for the cultivation of central nerve tissue the organ cavity is provided with three, four, five or six separate spaces for the maintenance for example of the different layers of grey matter of the cortex or cerebellum (from periphery to the center - granular cell layer, molecular cell layer and purkinje cell layer and the white matter layer formed by nerves). The three grey matter sections of this organ cavity are loaded with tissue sections of the respective parts of the brain or are filled with the respective neurons and mixed with the necessary amount of glia cells. The walls between the sections allow for dendrite and axon passages. Axon based nerves are located in the segment directly connected to the stem cell cavity (9) (see below) and, therefore, can penetrate through the upper part of the stem cell cavity (9) to other organ cavities (4). Impedance measurement means at the bottom of relevant segments can be provided and may serve as sensor to proof re-establishment of functional grey matter layer connection.

In a preferred embodiment of an organ cavity (4a) designed for the cultivation of a hybrid bone / cartilage organoid as present in joints the organ cavity is subdivided in a central bone area and a peripheral part representing the cartilage area. The cartilage area, which is preferably larger than the bone area, is loaded with collagen matrix, chondroblasts and chondrocytes and will be pressurized constantly or periodically by a pressurizing means integrated in the small niche in the periphery of this segment. This segment will be closed at its top preferably by way of the upper closing layer (14) fluid-tight with a foil or flexible sheet, which is preferably not permeable to oxygen. The interface to the central bone segment may be coated with bone growth factors like, e.g. bone morphogenic protein (BMP). The bone segment may be loaded preferentially with bone marrow specula's or calcified collagen matrix loaded with osteoclasts and osteoblast.

In a preferred embodiment of an organ cavity (4b) designed for the cultivation of a vascularised skin equivalent a micro-vessel in fluid-tight connection with two peripheral reservoirs and formed from biodegradable or synthetic polymers allow endothelial cells to confluently attach to the inner wall and to grow out into surrounding tissue. Between the reservoirs a pumping means is provided to circulate blood or blood substitutes through the vessel. The organ cavity may be filled with extracellular skin matrix and keratinocyte suspension and/or with tissue slices of skin. In addition hair follicles may be seeded into the segment, thus providing architecture and microenvironment to develope a vascularised skin equivalent in the organ cavity. Optionally, a degradable matrix or preassembled microchannel are provided which will be seeded by endothelial cells to form a capillary network within an organ cavity or between two or more or all organ cavities (4, 4a, 4b) within an organ growth section (3).

In a preferred embodiment the organ cavity is designed to support the formation of liver segments by providing a spaced environment which allow growth of liver segments with a maximal length of 500 µm, by providing a O₂ gradient across the entire liver segment and providing to the polar hepatocytes a "blood side" and a "gall side". Any gall that may be produced can be drained to a separate waste reservoir through a microchannel provided for that purpose. Thus, the liver segment will be supplied from the centre or the organ segment, while the waste including optionally separate gall disposing micrchannels will be located at the periphery. It is envisioned that pore containing collagen matrices or prestructured synthetic scaffolds are used to allow attachment of sinusoid cells and the formation of a disse gap. It is envisoned that the hepatocyte layers will be embedded in the organ cavity within a semi-solid or solid matrix. for optimal interaction with the endothelial cells and Kupffer-cells.

During operation of the organ-on-a-chip device two, three, four, five or more different tissues, organoids, or organs formed separately in the two, three, four, five or more organ cavities (4, 4a, 4b) within a growth section (3) may interact with each other. Interaction may occur between the organ cavities through, e.g. outgrowth of nerves from (4) and/or microcapillaries from (4b) into the other cavities. Such interaction may occur through separately provided connecting channels/openings between two different organ cavities (4, 4a, 4b), which may be opended or closed as desired and/or through the centrally located stem cell cavity (9). As already indicated above, it is preferred that capillaries allowing medium flow within an organ cavity (4, 4a, 4b) and/or within an organ growth section (3) are provided. To that end either preassembled non-degradable microchannels that can be populated with endothelial cells, or degradable matrixes, e.g from Matrigel, may be arranged in the organ growth section to connect two or more organ cavities. Endothelial cells will then grow using the guidance of the matrix. Alternatively, a synthetic cell free circulation network may be provided. In case of significant damage signals originating from the organoids and/or organs in the organ cavities (4, 4a, 4b) the quiescent stem cells in the stem cell cavity (9) comprising a hematopoetic stem cell niche, which may be formed at the bottom of the stem cell cavity (9) and has a restricted fluid flow, build up from osteoblast feeder cells and hematopoetic stem cells, may regenerate such damages for example in the bone and cartilage organ cavity.

The medium feed reservoir (2) holds the medium and/or supplements necessary to differentiate and/or maintain the cells in the organ growth sections. The size of the medium feed reservoir comprised in the self-contained organ-on-a-chip device of the present invention is determined by several parameters including: (i) required self-contained cultivation period and (ii) required medium change rate. Typically the medium feed reservoir comprises medium in excess of one organ cavity (4, 4a, 4b) volume per day of culture multiplied by the number of connected organ cavities and the number of culture days and, if required supplements. In a preferred embodiment the self-contained cultivation period is at least 10 days, 15 days, 20 days, 25 days, 30 days, 35 days 40 days, 45 days, 50 days, 60 days, 70 days, 80 days, or 90 days or more. Accordingly, the size of the medium feed reservoir (2) contained within the self-contained organ-on-a-chip device of the present invention can be calculated on the basis of the following formula: (nₒ · vₒ · Xₘ · t_{c}), wherein nₒ indicates the number of organ cavities,· vₒ indicates the volume of the organ cavities (on the assumption that the volume of all organ cavities is similar, i.e. ± 20%, otherwise the individual volumes of the organ cavities have to be added up), Xₘ indicates the medium exchange rate per day and t_{c} indicates the self-contained cultivation period. Preferred values for no are between 18 to 96, for vₒ between 0.5 to 2 mm³, for Xₘ are between 0.5 to 2 and for t_{c} between 14 to 90. Typically the medium waste reservoir has at least a volume corresponding to the volume of the medium feed reservoir. In a typical embodiment the medium feed reservoir (2) comprised within the self-contained organ-on-a-chip device has a volume of between 2 ml to 5 ml. Given this rate of fluid flow, there is typically no necessity to provide a venting system to the medium feed reservoir (2), to avoid negative pressure build up, since any required gaseous medium is capable to diffuse through the gaseous permeable membrane covering the organ cavities into the organ cavities and back into the medium feed reservoir (2). The lack of a venting system is a preferred embodiment since it minimizes the risk of contamination of the medium feed reservoir. Depending on the type of cells, tissues, organoids or organs to be established and/or to be maintained in the organ growth section (3) one type of medium will be sufficient to support differentiation and/or maintenance of all cells, tissues, organoids or organs or it may be required to provide different media to different organ growth sections (3) and/or different media to different organ cavities (4, 4a, 4b) within one organ growth section (3). It may also be required to provide two or more different media at different points in time, e.g. during differentiation and maintenance, respectively. Thus, the organ-on-a-chip device (1) may comprise in certain embodiments 2, 3, 4, 5, 6, 7, or more different medium feed reservoirs (2), which are in fluidic communication with an organ growth section through a microfluidic feed channel (6). As some cells, tissues, organoids or organs may require a second medium one medium feed reservoir (2) may be in fluidic communication with only one organ cavity (4, 4a, 4b) within a given organ growth section (3), which is designed to provide a microenvironment for a cell type requiring such a second medium.

Preferably, at least one microfluidic feed channel (6) fluidically connects the medium feed reservoir (2) with the one or more organ growth sections (3). The diameter of the microfluidic feed channels is preferably between 100 nm to 1 mm, preferably between 0.5 µm to 200 µm, more preferably 1 µm to 100 µm It is preferred that the microfluidic feed channel (6) is provided with a further outlet, which allows the administration of supplements and/or test compounds to the organ growth sections (3) separately. It is preferred that such an outlet is positioned in a sufficient distance from the outlet of the microfluidic feed channel (10) to allow mixing of the medium and the supplements and/or test compound to ascertain even distribution of the supplements and/or the respective test compound between two or more organ cavities within one organ growth section.

To control the flow of medium and/or supplements to each organ growth section it is possible to provide a flow control means in the flow path from the medium feed reservoir (2) to the organ growth sections (3). Such control of flow is implemented preferably by external pressure sources, external mechanical pumps, integrated mechanical micropumps, or by electrokinetic mechanisms. Process monitoring capabilities in continuous-flow systems can be achieved with highly sensitive microfluidic flow sensors based on, e.g. MEMS technology, which offer resolutions down to the nanoliter range. Thus, such devices may also be present in the flow path either to the organ growth section and/or from the organ growth section.

In a preferred embodiment the self-contained-organ-on-a-chip device (1) of the present invention further comprises at least one stem cell cavity (9), preferably a neonatal, pre/postnatal and/or adult stem cell cavity within the organ growth section (3). The stem cell cavity is a microstructured region within the organ growth section (3) that provides an environment that is suitable for maintenance of stem cells at different stages of differentiation. Thus, stem cells may migrate independently into the stem cell cavity (9) or may be directly introduced into the stem cell cavity (9) either together or independently from the cells introduced into the one ore more organ cavities comprised within the organ growth section (3). Guiding elements for formation of stem cell cavities (9) *in vivo* are dimension, shape, surface properties (e.g. feeder cells), nutrition characteristics and fluidic profile. Preferably the stem cell cavity is fluidically connected to the one or more organ cavities (4, 4a, 4b) to allow stem cells to migrate into the various organ cavities (4, 4a, 4b) to aid regeneration and maintenance of the respective organoid, and/or organ. It is preferred that the stem cell cavity is fluidly connected to the at least one organ cavity (4, 4a, 4b) by an opening of less than 80 µm but larger than 10 µm. Preferably, the stem cell cavity (9) has a diameter of between 10 to 200 µm, preferably less than 100 µm. To allow stem cells comprised in the stem cell cavity similar access to two or more organ cavities comprised in the organ growth section (3) it is preferred that the stem cell cavity (9) is located equidistantly to the organ cavities (4, 4a, 4b). In a preferred embodiment, wherein the organ growth section (3) comprising two, three, four, five, six or more organ cavities (4, 4a, 4b) has a circular shape it is preferred that the stem cell cavity (9) is disposed in the center of the organ growth section (3). It is further preferred that the stem cell cavity (9) is arranged opposite the outlet (10) of the microfluidic feed channel (6), which allows preferred access of stem cells in the stem cell cavity to fresh medium. Preferably the stem cell cavity is lined with a basal lamina as matrix. Such lamina may be produced at the interphase of epithelial cells and fibroblast and, thus, may be provided through *de novo* synthesis in a first step of establishing the stem cell cavirty or may be derived from decellularized tissue known to comprise basal lamina and may be introduced into the stem cell cavity prior to seeding with stem cells. Such a basal lamina is supportive of the attachment and maintenance of stem cells in the stem cell cavity, preferably a prenatal, postnatal and/or adult stem cell cavity.

A neonatal stem cell cavity is one that provides an environment suitable for attracting/maintaining neonatal stem cells. A preferred embodiment of the neonatal stem cell cavity promoting neonatal development is a hollow body, preferably a hollow cylinder. This hollow body, e.g. cylinder, preferably has a height of between 200 µm to 1,000 µm, preferably 400 µm and a diameter of 80 µm to 300 µm preferably a diameter of 100 µm. It is preferred that only one organ growth cavity (4, 4a, 4b) is in fluidic connection with that stem cell cavity. Preferably the outlet (10) of the microfluidic feed channel (6) and the fluidic connection to the organ growth section(s) are positioned in such that the medium flows through the hollow cylinder over the stem cells that are located in the stem cell cavity. If the cavity is within a circular organ growth section it is preferred that it covers less than 10%, preferably less than 2.5% of the surface area of the organ growth section (3). Preferably the fluidic connection to the organ cavity (4, 4a, 4b) is at a side of the stem cell cavity (9) opposite to the outlet of the microfluidic feed channel (6). Preferably, the cylinder is connected with the conical organ cavity only in the lower part of the cylinder - with respect to the outlet (10) of the microfluidic feed channel (6). This opening preferably has a height of about 150 to about 450 µm, preferably about 300 µm from the bottom of the stem cell cavity, which equates to the approximate diameter of the embryonic gastrula undergoing asymmetric division. Preferably the neonatal stem cell cavity does not comprise a coating, a matrix, and/or a feeder layer. Blastula and gastrula formation in the stem cell cavity will occur within several days by symmetric stem cell division. Preferentially, the stem cell cavity may be filled with yolk sac medium for this purpose. This medium may be provided from the medium feed reservoir (2) or a second medium feed reservoir, if this medium is only used for establishing the organ, and/or organoid or may be introduced into the stem cell cavity, when seeding neonatal stem cells into the cavity. If the yolk sac medium is provided from a medium feed reservoir (2), perfusion in time with asymmetric division will guide tissue development into the organ cavity (4, 4a, 4b), preferably the single organ cavity. The organ cavity preferably has a length of about 3 mm. Organ growth sections (3) comprising neonatal stem cell cavities and tissues, organs and organoids developed therefrom are preferably used in basic research, preferably in developmental biology across all kingdoms of multi-cellular organisms and for embryotoxicity testing of substances

A pre- and postnatal stem cell cavity is one that provides an environment suitable for attracting/maintaining pre-and postnatal stem cells, which promote maturation and fast growth of organs and tissues during pregnancy and childhood. A preferred embodiment of a pre- and postnatal stem cell cavity is a hollow body, preferably a cylinder with an area with a reduced fluid flow. The fluid flow in that area - which may also be referred to as stem cell niche - is reduced with respect to the fluid flow in other parts of the stem cell cavity (9). Thus, the majority of medium will flow into and out of the stem cell cavity (9) into the organ cavities (4, 4a, 4b) without entering this particular area. The height of the hollow body, preferably cylinder, is between 200 µm to 1,000 µm, preferably 400 µm. The diameter is preferably between 10 µm and 300 µm preferably the diameter is between 10 µm to 200 µm, e.g. 20, 30, 40, 50, 60, 70, 80, 90, 100 110, 120, 130, 140, 150, 160, 170, 180, 190, or 200 µm. It is preferred that several, i.e. two, three, four, five, or more, preferably identical organ growth cavities (4, 4a, 4b) are in fluidic connection with the pre- and postnatal stem cell cavity. Preferably the fluidic connection to the one or more organ cavities (4, 4a, 4b) is positioned in the middle of the hollow body, preferably in the middle of the cylinder. This/these opening(s) preferably has/have a distance from the bottom of the stem cell cavity - with respect to the outlet (10) of the microfluidic feed channel (6) - of about 100 to about 300 µm, preferably about 200 µm, thereby forming an area at the bottom of the stem cell cavity with a reduced flow of medium, which is preferable for maintenance of pre- and postnatal stem cells in the stem cell cavity (9). The openings are preferably slit-like. Thus in a preferred embodiment the medium will flow through the outlet (10) of the microfluidic feed channel (6) into the top of the stem cell cavity (9) and out into the organ cavities in the middle section of the stem cell cavity (9). The area of reduced flow is located in the lower part of the hollow part, preferably cylinder. To establish organ specific pre- and postnatal stem cell niches in this area, they are preferably composed of a stem cell niche specific feeder cell layer, adhered to the surface of the lower part of the cavity or to microporous microcarrier material of biological or synthetic origin. The corresponding stem cells are transferred into the stem cell cavity, preferably directly to the bottom of the stem cell cavity and cultured under constant conditions, e.g. using prenatal or new born serum media flow. In the lower part of the niche at the bottom of the niche, where medium flow is minimal and nutrient supply is mainly provided by diffusion, symmetric stem cell divisions ensures stem cell self-renewal. As cells growth up to larger tissue clusters, nutrient gradients and media flow turbulences appear in higher niche regions, supporting asymmetric division and outflow of organ progenitor cells into identical organ cavities (4, 4a, 4b) of a defined size, shape, microenvironment and architecture. Organ growth sections (3) comprising pre- and postnatal stem cell cavities and tissues, organs and organoids developed therefrom are preferably used in basic research, preferably in research on organ maturation and functionality of stem cell niches during pre- and postnatal life. Furthermore they are preferably used for toxicology, pharmacodynamic or pharmacokinetic of substances with regard to their biosafety or mode of action preferably during childhood.

An adult stem cell cavity is one that provides an environment suitable for attracting/maintaining adult stem cells. A preferred embodiment of an adult stem cell cavity promoting formation of adult quiescence-promoting stem cell niches of discrete organs, is a hollow body, preferably a cylinder with an area with a reduced fluid flow. The fluid flow in that area - which may also be referred to as stem cell niche - is reduced with respect to the fluid flow in other parts of the stem cell cavity (9). Thus, the majority of medium will flow into and out of the stem cell cavity (9) into the organ cavities (4, 4a, 4b) without entering this particular area. The height of the hollow body, preferably cylinder, is between 200 µm to 1,000 µm, preferably 400 µm. The diameter is preferably between 10 µm and 300 µm preferably the diameter is between 10 µm to 200 µm. It is preferred that several, i.e. two, three, four, five, or more, preferably different organ growth cavities (4, 4a, 4b) are in fluidic connection with the adult stem cell cavity. Preferably the fluidic connection to the one or more organ cavities (4, 4a, 4b) is positioned in the middle of the hollow body, preferably in the middle of the cylinder and extends to the top of the stem cell cavity, preferably in a slit-like fashion. The opening(s) preferably have a distance from the bottom of the stem cell cavity - with respect to the outlet (10) of the microfluidic feed channel (6) - of about 100 to about 400 µm, preferably about 300 µm, thereby forming an area at the bottom of the stem cell cavity with a reduced flow of medium, which is preferable for maintenance of adult stem cells in the stem cell cavity (9). Thus in a preferred embodiment the medium will flow through the outlet (10) of the microfluidic feed channel (6) into the top of the stem cell cavity (9) and out into the organ cavities without entering the bottom section of the stem cell cavity (9). In preferred embodiments but not limited thereto a follicular bulge stem cell niche of skin, a crypt base columnar stem cell niche of small intestine, a bronchoalveolar stem cell niche of lung, a hematopoietic stem cell niche for blood reconstitution, a sub-ventricular zone stem cell niche for regeneration of nerve tissue or a stem cell niche for maintenance of hormone glands will be formed within the stem cell cavity (9). Depending on the particular combination of organoids and organ systems, the managing of centralized organ nutrition and regulation will lead to the formation of auxiliary structures to the organs and organoids, including nerves and blood vessels that may extend from one organ cavity (4, 4a, 4b) into another organ cavity (4, 4a, 4b), preferably by crossing through the stem cell cavity (9).

Organ specific adult quiescence-promoting stem cell niches are established in the adult stem cell cavity (9) preferably by introducing feeder cells, matrices and stem cells in the stem cell cavity (9) specific for the stem cell niche to be established, preferably into the lower part of the cavity (9). An overview of components composing the adult physiological stem cell niches of different organs is given by D.L. Jones and A.J. Wagers: No place like home: anatomy and function of the stem cell niche. Nature Reviews/ Molecular Cell Biology, V.9, pp. 11-21, January 2008, which is incorporated in its entirety by reference, in particular with respect to its teaching on the requirements (e.g. coatings, growth factors, extracellular matrix components) for the establishment of the respective stem cell cavity..

Adhesion molecules which may help maintaining the stem cells within the stem cell cavity (9) can be selected from integrins, catenins, cadherins, other cell adhesion proteins, or combinations thereof. Adhesion molecules suitable for maintaining the stem cells within the stem cell cavity (9) may preferably be selected from α6 integrin, β1 integrin, β-catenin, E-cadherin, N-cadherin, or combinations of two or more of said proteins but are not limited thereto. The stem cell cavity (9) may also comprise cells that act as support cells for stem cells. Suitable support cells may be selected from the following list of cell types but are not limited to these cell types: osteoblasts, vascular cells, crypt fibroblasts, Paneth cells, dermal fibroblasts, vascular cells, astrocytes, Sertoli cells, interstitial cells, and combinations of two or more of said cells. The skilled person will be aware that the selection of the suitable support cell(s) depends on the stem cell to be maintained in the stem cell cavity (9). In particular, osteoblasts are suitable as support for haematopoietic stem cells (HSCs); vascular cells are suitable as support for HSCs, subventricular zone (SVZ) stem cells, subgranular zone (SGZ) stem cells, and spermatogonial stem cells (SSCs); crypt fibroblasts as well as Paneth cells are suitable as support for crypt base columnar cells (CBCs); dermal fibroblasts are suitable as support for follicular bulge stem cells; astrocytes are suitable as support for SVZ stem cells and SGZ stem cells; and Sertoli cells as well as interstitial cells are suitable as support for SSCs. It is further contemplated that the mechanical properties of the stem cell cavity (9) influences stem cell function. In particular, the relative elasticity or stiffness of the stem cell cavity (9) can directly modify stem cell differentiation decisions (D.L. Jones and A.J. Wagers (2008), supra). For example, a relatively elastic substrate may be used in the stem cell cavity (9) to promote neural differentiation of mesenchymal stem cells (MSCs). In contrast, choosing a rigid substrate in the stem cell cavity (9) will favour osteoblast differentiation of MSCs. Finally, a substrate of intermediate stiffness will prompt differentiation into the skeletal muscle lineage. It is further contemplated to add one or more factors which influence stem cell maintenance, differentiation and/or quiescence to the stem cell cavity (9). For example, osteopontin (OPN) suppresses stem cell expansion in the HSC niche (W.P. Daley et al.: Extracellular matrix dynamics in development and regenerative medicine. J. Cell Science (2008) v. 121, pp. 255-264) and may be added to the stem cell cavity (9) for the same effect. Other exemplary factors that may be usable are steel factor (SLF), Wnt, Notch, angiopoietin-1 (ANG1), bone morphogenetic protein (BMP), sonic hedgehog (Shh), and glial cell-line-derived neurotrophic factor (GDNF).

It is preferred that the components are embedded into a semisolid medium, preferably agarose, methyl cellulose or alginate. Preferably, the organ cavities are loaded with the matrices, coatings, cell suspensions or cell clusters such as tissue slices, which are respectively required for the formation of the desired organoid, and/or organ. Preferably a constant flow of medium, e.g. comprising adult serum or a synthetic complete medium, is provided. The amount of the flow is such that it does not disturb the stem cell niche which may form at the bottom of the stem cell cavity (9). Due to shape and geometry it is preferred that the adult stem cell niche is provided with nutrients exclusively by diffusion. Once vascularization or nerve growth takes place in one or another organ cavity, nerves and microcapillaries can easily penetrate the other organ cavities through the upper part of the stem cell niche, thus innervating or vascularizing other organoids of the same organ growth section. Once the whole system has reached natural homeostasis, test substances can be applied. The cells of the stem cell niche are predominantly quiescent and may be activated to regenerate organ segments only when receiving damage signals from the organ cavities. Organ growth sections (3) comprising one or more adult stem cell cavities and tissues, organs and organoids developed therefrom are preferably used in basic research, preferably in research on adult stem cell niches, organ physiology and homeostasis. Furthermore they are preferably used for testing of substances relevant to consumer health.

It is further preferred that the stem cell cavity (9) is cylindrical. This is a particularly preferred shape, if the stem cell cavity (9) is located in the center of the organ growth section (3).

In a second aspect the present invention relates to an organ-on-a-chip device (1), preferably a self-contained organ-on-a-chip device, comprising
(a) at least one organ growth section (3) comprising at least one organ cavity (4, 4a, 4b), and
(b) wherein the at least one organ cavity (4, 4a, 4b) comprises and/or is connected to at least one sensor (8, 8a, 8b). Preferably the self-contained organ-on-a-chip device (1) according to the second aspect, further comprises at least one medium feed reservoir (2) wherein the medium feed reservoir (2) is connected to the at least one organ growth section (3) by a microfluidic feed channel (6) and the at least one organ cavity (4, 4a, 4b) comprised therein. All terms used with respect to the second aspect, e.g. "self-contained", "organ section (3)", "organ cavity (4, 4a, 4b)" and "medium feed reservoir" have the meaning and preferred meanings as outlined above and the term "sensor" has the meaning as outlined below. It is further preferred, that the organ section (3) further comprises at least one, preferably one stem cell cavity (9), preferably a neonatal, pre/postnatal or adult stem cell cavity.

In a third aspect the present invention relates to an organ-on-a-chip device (1), preferably a self-contained organ-on-a-chip device (1) comprising:
(a) at least one organ growth section (3) comprising at least one organ cavity (4, 4a, 4b), and
(b) wherein the organ growth section (3) comprises a stem cell cavity (9).

In the context of this third aspect the terms used, e.g. "organ growth section", "organ cavity" and "stem cell cavity" have the meaning and preferred meanings indicated for the first aspect of the present invention. The provision of a stem cell cavity (9) having the properties outlined above within the organ growth section (3) provides an improved culturing system for tissues, organoids and organs maintained in the organ cavities (4, 4a, 4b), since it simulates the natural situation, wherein organs and tissues are replenished by stem cells, which are keeping dormant but proliferation competent within or in the vicinity of the organ and/or tissue.

The organ-on-a-chip device (1) according to the third aspect of the present invention preferably comprises at least one medium feed reservoir (2), wherein the medium feed reservoir (2) is connected to the at least one organ growth section (3) by a microfluidic feed channel (6). Again in this context the terms "medium feed reservoir (2)" and "microfluidic feed channel (6)" have the same meaning and preferred meanings outlined above with respect to the first aspect of the invention.

It is possible that a separate medium waste reservoir is provided that is attached through a connector to the organ-on-a-chip device (1) according to any aspect of the present invention, i.e. is a separate entity. In this case any waste medium will be removed from the device and can be disposed of, while the device is operated. It is, however, preferred that the organ-on-a-chip device according to any aspect of the present invention further comprises at least one medium waste reservoir (5), wherein the at least one organ cavity (4, 4a, 4b) is connected to the at least one medium waste reservoir (5) by a microfluidic waste channel (7). In this preferred embodiment the entire fluid provision and disposal requirements are contained within the organ-on-a-chip device of the present invention, which further increases the flexibility and decreases the risk of contamination. While each organ growth section (3) including all organ cavities (4, 4a, 4b) comprised therein may be connected through one microfluidic waste channel (7, 7a,7b), it is preferred that each organ cavity is separately connected to a medium waste reservoir (5) specifically provided for that organ cavity (4, 4a, 4b).

The properties of the cells, tissues, organoids and/or organs established and/or maintained in the organ growth section and organ cavities, respectively, can be monitored in the medium flow through drained from the organ growth section (3) and organ cavities (4, 4a, 4b), respectively, or within the organ cavity (4, 4a, 4b). Such properties may comprise secreted or released substances, modified substrates, change of impedance, electric pulses, mechanical forces etc. To detect these properties it is preferred in either aspect of the organ-on-a-chip device (1) of the present invention that at least one sensor (8, 8a, 8b) is arranged between the at least one organ cavity (4, 4a, 4b) and at least one medium waste reservoir (5) and/or within the at least one organ cavity. Such sensors (8, 8a, 8b) are known in the art and are preferably selected from the group consisting of pH sensor; pO₂ sensor; analyte capture sensor; surface acoustic wave sensor (SAW), sensor; plasmon resonance sensor; temperature sensor; CO₂ sensor; NO sensor; chemotaxis sensor; cytokine sensor; ion sensor; potentiometric sensor; amperometric sensor; flow-through-sensor; fill sensor; impedance sensor; conductivity sensor; tension sensors, electromagnetic field sensor; and metabolic sensor. The property of the cells, tissues, organoids and/or organs that may be assessed will depend on the respective cells, tissues, organoids and/or organs. Thus, it is envisioned that different sensors are provided for different cells, tissues, organoids and/or organs comprised in separate organ cavities within one organ growth section (3). For electrically active cells, organoids and organs multi-microelectrode arrays represent a powerful technique. A. Robitzki et al: Cells on a chip - the use of electric properties for highly sensitive monitoring of blood-derived factors involved in angiotensin II type 1 receptor signaling. Cell Physiol. Biochem. 16 (1-3), 51-58 2005. For electrophysiologically inactive cells and organoids impedance spectroscopy, as described in J. Aguilo et al: Impedance dispersion width as a parameter to monitoring living tissues. Physiol. Meas. 26 (2), 165-173, 2005, should be applied. Alternatively or additionally two or more, e.g. two, three, four, or five different sensors are provided within the flow path either to provide a system with an increased flexibility or to monitor two or more properties simultaneously. If, for example, the capability of a liver organoid is tested to metabolize a given substance it may be required to determine the amount of metabolite in the flow through and any apoptosis or necrosis, which may occur in the organoid. Live cell, organoid or organ imaging combined with two photon microscopy penetrating tissues to a depth of more than 1 mm thickness can be applied to any stem cell or organ cavity within the organ on a chip device (1). In a further preferred embodiment the self-contained organ-on-a-chip device further comprises a temperature sensor arranged to determine the temperature in the at least one medium feed reservoir (2) and/or the at least one organ cavity (4, 4a, 4b).

Alternatively or additionally sensing substances, e.g. pH sensory substances to the medium and flow with the medium. Preferably such ensory substances may already be comprised in the medium feed reservoir (2), may be comprised in a separate reservoir and may be added continuously, at predetermined intervals or when required to carry out certain measurements ormay be added through the membrane or flexible sheet directly into the organ growth section (3), preferably directly to the organ cavity (4, 4a, 4b). Typically, such sensing sustances alter a chemical and/or physical property in response to a change in the environment, e.g. pH, pO₂, salt concentration, temperature, presence or absence of an analyte etc. Such an alteration of a physical property may be, e.g. a change in absorption or emission property, e.g. fluorescence, or change of redox-potential of the sensing substance. In some preferred embodiments such sensing substances may be immobilized within an organ cavity (4, 4a, 4b) or may be immobilized on or within a microbead or nanobead. For the purpose of the present invention the term "microbead" refers to a preferably circular particle with a diameter of between 20 µm to 0.5 µm and the term "nanobead" referst to a preferably circular particle with diamenter of lower than 0.5 µm. Based on the dimension of such beads they may flow with the medium or may remain with an organ cavity. To affect movement of microbeads or nanobeads within the organ cavity (4, 4a, 4b), preferably of those beads that are to large to be carried along with the medium flow, it is envisioned that beads are pro vided with a magnetic or magnetizisable core, that may be moved by magnetic or electric fields generated in the organ-on-a-chip device. Such beads may also be added through the opening on top of the growth segment, which may be resealed thereafter or may provide such resealing through the flexible sheet covering the opening.

To provide an appropriate environment for the organoid or organ to be established and maintained the organ cavity (4) comprises one or more structures selected from four types, namely, microstructures, chemical modifications structures, actuating means, and sensory means or combinations thereof. Typically the organ cavity comprises tissue specific microstructures and chemical modification structures.

The "microstructures" comprise, e.g. three-dimensional, preferably biodegradable polymer scaffolds, which are provided with the goal of inducing the correct type of cells from, e.g. the stem cell cavity, to migrate into the organ cavity or to provide additional cell attachment surface areas or microenvironments within an organ cavity (4, 4a, 4b). The microstructures may be biodegradable, meaning that over time they will break down both chemically and mechanically. As this break down occurs, the cells secrete their own extracellular matrix, which plays a critical role in cell survival and function. In normal tissue, there is an active and dynamic reciprocal exchange between the constitutive cells of the tissue and the surrounding extracellular matrix. Latest discoveries in this field are summarized in W.P. Daley et al: Extracellular matrix dynamics in development and regenerative medicine, Journal of Cell Science, 121, 255-264. The extracellular matrix provides chemical signals that regulate the morphological properties and phenotypic traits of cells and may induce division, differentiation or even cell death. In addition, the cells are also constantly rearranging the extracellular matrix. Cells both degrade and rebuild the extracellular matrix and secrete chemicals into the matrix to be used later by themselves or other cells that may migrate into the area. It has also been observed that the extracellular matrix is one of the most important components in embryological development. Pioneering cells secrete chemical signals that help following cells differentiate into the appropriate final phenotype. For example, such chemical signals cause the differentiation of neural crest cells into axons, smooth muscle cells or neurons. Microstructures preferably comprise micro-carriers, preferably collagen micro-carriers; calcification zones, preferably calcified collagen; synthetic polypeptide gels, preferably polyaminoacid gels, e.g. glutamine gels capillaries that may extend through the organ cavity, and may be connected to further medium feed reservoir(s); and woven and/or non woven polymeric hollow fibres, preferably polyethersulfone or polylactid fibres. Such microstructures are preferably introduced into the organ cavity once the organ-on-a-chip device has been at least partially or has been completely assembled. Thus, microstructures are preferably separate from the material forming the organ cavity, which may, as outlined above provide additional substructures like ridges, channels, or funnels etc.

The term "chemical modifications structures" as used herein relates to substances, which are adhered, e.g. absorbed, covalently or non-covalently attached, to all or part of the surface of the organ cavity (4, 4a, 4b) in thin layers, typically in monomolecular layers. Preferred examples comprise peptides, proteins like, e.g. bone morphogenic protein (BMP), neuronal growth factor, erythropoietin, colony stimulating factors, interleukins, interferons, integrins, selectines or receptors of above mentioned proteins and cross-linked proteins, preferably RGD motif comprising peptides, or proteins e.g. albumins, transferrins, insulins or fibrins. For cross-linking of proteins a variety of art known cross-linking agents can be used comprising glutaraldehyde. For local attachment to a part of the cavity photochemical sensitization of the surface can be applied.

Actuating means are provided, preferably within the organ cavity (4, 4a, 4b) to more completely simulate the natural environment, which in addition to chemical cues will also provide physical cues that are required for establishment and maintenance of specific tissues, organoids and/or organs. Thus, such actuating means comprise means that change the physical state of the cells by exerting pressure on the cell mass as required, e.g. for bone and cartilage formation, pump fluids back and forth in parts of the organ cavity to simulate capillary blood flow or a tissue interface as found in the gut, provide heat or electric stimulation. Such actuating means preferably comprise: at least one pulsative pressurizing means located in a separate sub cavity of a given organ cavity for providing a secondary flow through the organ cavity, one or more electrodes, electromagnetic field forces, or micropumps, including piezo elements, elastic membranes that swing back and forth, elastic hollow spheres seeded with pacemaker cells/cardiomyocytes that twitch termed "microheart", surface acoustic wave engines (SAW) or magnetic pistons that act on membranes within the organ cavities.

Alternatively, single cells or organ parts may be moved within an organ cavity or into or out of an organ cavity by using beads with magnetic or magnetizisable core and ligands attached to the surface that preferentially bind to certain cell types or groups of cells and by applying magnetic or electric fields. Suitable beads are well known to the skilled person and come either as micro or nanobeads. The choice of the bead type will be determined by the number of cells that are to be moved when applying the electric or magnetic field and, thus, both micro and nanobeads may be used. The self-contained organ-on-a-chip device may already be assembled with a certain amount of such beads preloaded or may be loaded, preferably through the opening of the organ cavities, with the beads as required.

To assess the state of differentiation and health or a number of other properties comprising metabolic activity, number of apoptotic cells, number of proliferating cell etc. of the developing or developed organoids or organ sensory means are provided in the organ cavity (4, 4a, 4b) thereby allowing a more direct access of the sensors to the cells, if compared to sensors that are located, e.g. in the flow path to the microfluidic waste compartment (8, 8a, 8b). Preferred sensors, comprise temperature sensors, sensor substances, which are preferably attached to the surface of the organ cavity or cytokine specific antibodies coupled on multiple microsurfaces, made from gold, positioned in the outlet waste channel and observable by means of Plasmon resonance, and optical fibres, which allow to provide light of different wavelengths to the organ cavity and detect any light, which may be emitted, reflected or adsorbed in the organ cavity. Sensor substances are substances that change a measurable physical or chemical property in response to a given cue. As outlined above such surfaces may also be the surface of a microbead or nanobead that is positioned within the organ cavity (4, 4a, 4b).

Specific combinations of substructures within the organ cavity, microstructures, chemical modifications structures and actuating means provide an environment for establishment and differentiation of particular tissues.

Organ cavities (4, 4a, 4b) within an organ growth section (3) provide space for self-assembly, maintenance and/or re-assembly of the smallest functionally self-reliant structural unit of a specific organ (e.g. Alveoli of Lung, Epidermis and Dermis of Skin, Gut Mucosa, Liver Lobulus, Nephron of Kidney,) or a specific organ system (e.g. microvasculature of Blood System, gray matter of Nerve System). Nature's principle building blocks for directed organ assembly *in vivo* are dimension, shape, nutrition characteristics, micro-architecture (e.g. extracellular matrix & membranes, surfaces properties) and local microenvironment (e.g. morphogen and chemokine gradients). A preferred embodiment of organ cavities is provided exemplarily for three types of organs (4) - brain tissue, 4a - bone-cartilage and 4b-vascularized skin), setting proper dimension, shape and nutrition for each specific organ and providing access to introduce additionally necessary elements of micro-architecture and micro-environment as well as to load the organ on a chip device with the cell suspension or tissues.

In a preferred embodiment, organ cavity (4), for example, is designed for the cultivation of central nerve tissue, providing four separate spaces for the maintenance for example of the different layers of gray matter of the cortex or cerebellum (from periphery to the center-granular cell layer, molecular cell layer and purkinje cell layer and the white matter layer formed by nerves). The three gray matter sections of this organ cavity are loaded with tissue sections of the respective parts of the brain or are filled with the respective neurons and mixed with necessary amount of glia cells. The walls between the section allow for dendrite and axon passages. Axon based nerves are located in the segment directly connected to the stem cell niche and therefore can penetrated through the upper part of the niche to other organ cavities. Impedance measurement means at the bottom of relevant segments serve as sensor to proof re-establishment of functional gray matter layer connection.

In a preferred embodiment cavity (4a) provides dimension, shape and nutrition characteristics for a hybrid bone / cartilage organoid. The central smaller segment is the bone area and the larger peripheral part represents the cartilage area. The cartilage area is loaded with collagen matrix, chondroblasts and chondrocytes and will be pressurized constantly or periodically by a pressurizing mean integrated in the small niche in the periphery of this segment. This segment will be closed at top fluid-tight with a foil, not permeable for oxygen. The interface to the central bone segment will be coated with bone morphogenic protein (BMP). The bone segment will be loaded preferentially with bone marrow specula's or calcified collagen matrix loaded with osteoclasts and osteoblast.

In a preferred embodiment organ cavity (4b) contains a micro-vessel, fluid-tight connected with two peripheral reservoirs and formed from biodegradable or synthetic polymers allowing endothelial cells to confluently attach to the inner wall and to be able to growth out into surrounding tissue. Between the reservoirs a pumping mean is provided to circulate blood or blood substitutes through the vessel. The organ cavity is filled with extracellular skin matrix and keratinocyte suspension or with tissue slices of the skin. In addition hair follicles can be seeded into the segment, thus providing architecture and microenvironment to develop vascularized skin equivalent in the organ cavity.

Finally, operating such a growth segment, interaction may occur between the organ cavities through outgrowth of nerves from 4 or microcapillaries from 4b into the other cavities. In case of significant damage signals the quiescent stem cells in the for example hematopoietic stem cell niche, build up from osteoblast feeder cells and hematopoietic stem cells, may regenerate such damages for example in the bone and cartilage organ cavity.

While it is possible to assess several properties of the cells comprised in the organ cavities (4, 4a, 4b) indirectly by sensors as described above, it is also preferred that the self-contained organ-on-a-chip device (1) of the present invention is microscopable. To the end every part of the organ-on-a-chip device may be manufactured from an optically transparent material. Preferably one or more organ section (3), one or more organ cavities (4, 4a, 4b) and/or at least one sensor (8, 8a, 8b) are microscopable. Live cell imaging applying two photon microscopy through the whole organ cavity layer (15) provides online information on organs assembly and maintenance as well as effects of substances on organ behaviour. As set out above the organ-on-a-chip device comprises in a preferred embodiment an opening to the environment for each organ section (3). This opening is preferably of identical size as the organ section (3) itself. In operation it may be covered with a translucent, fluid tight, gas permeable material like, e.g. a spray on band-aid, a flexible sheet made from, e.g. PDMS, or a fibrin sheet. In this arrangement it is possible to directly observe the cells in the organ cavity by optical microscopy, e.g. by supplying light from above as in laser scanning-microscopy. A less sophisticated method that also provides high resolution images uses transmission microscopy. The use of transmission microscopy, however, requires providing optical transparent material along the entire light path through the organ-on-a-chip device (1) of the present invention. As will be explained in more detail below the microstructured layers used to assemble the organ-on-a-chip device of the present invention are preferably made of optical translucent materials like glass or SiO₂ and, thus, will be suitable to provide optical transparent material along the light path. If, for example, certain structures like heating elements are placed within the light path they may also be manufactured from optical translucent materials as, e.g. heating means manufactured from indium tin oxide (ITN). It is particularly preferred that the stem cell cavity (9) is microscopable to ascertain the occupancy and status of the cells in this cavity.

The flow of fluids through the microfluidic system of the self-contained organ-on-a-chip device (1) of the present invention may be achieved by, e.g. gravity or by capillary forces. To ascertain the flow of medium through the system it is, however, preferred that the medium waste reservoir (5) comprises a hydrophilic material, which once wetted will absorb the medium and, thus, provide a suction that is suitable to provide a fluid flow. Alternatively a micro pump may be arranged in the flow path between the medium feed reservoir (2) and the medium waste reservoir (5). The latter embodiment is advantageous in that the speed of the fluid flow can be adapted more easily to the growth conditions, cell numbers etc. observed in the organ growth section. Thus, if the apoptosis rate increases and/or the proliferation rate decreases the flow of medium may be increased to provide a better nutritional supply to the cells.

As has been set out above, it is preferred that all devices required for establishment and maintenance of tissues, organoids or organs are integrated within the self-contained organ-on-a-chip device (1) of the present invention. The advantage provided is the independence of the self-contained organ-on-a-chip device from secondary support units and medium supply and preferably disposable means. Thus, in a preferred embodiment the self-contained organ-on-a-chip device (1) also comprises heating means, arranged to either heat the at least one medium feed reservoir (2), the at least one organ cavity (4, 4a, 4b) or both. Preferably, these sections of the organ-on-a-chip device are heated to a temperature equivalent to temperatures found in the organism of which the cells are derived, e.g. 37°C. The heating means (11) may consist of any art known material that can form relatively thin, e.g. 1 to 100 µm heating elements. Preferred heating means are manufactured from indium tin oxide, platinum, gold or mixtures thereof. Out of these indium tin oxide is preferred due to the fact that it is optically translucent.

To allow an efficient manufacturing of the self-contained organ-on-a-chip device (1) of the present invention, it is preferably assembled of two, three, four, five, six, seven or more separately manufactured layers, depending on the required complexity of the microstructures. These layers can be manufactured by a variety of methods comprising machining from solid blocks of material, by e.g. milling, or laser ablation; casting, or optical lithography techniques as commonly used in the field of semi-conductors. Structures that are on the surface of one layer may become an internal closed structure once a second layer with corresponding microstructures is connected with the first layer in a fluid-tight manner. It is preferred that all layers have the same length and width to ascertain that once all layers are connected to each other to form a chip device that appears as a monolithic block. Thus, in a preferred embodiment the device of the present invention comprises or consists of a medium layer (12) and an organ growth section layer (13). In a preferred embodiment both layers are preassembled and delivered to the point of use as one monolithic chip, which may or may not already comprise medium in the medium feed reservoir (2) and/or supplements. It is, however, envisioned that in some embodiments the medium layer (12) is provided separately from the organ growth layer (13) and may only be attached to the organ growth layer once the cells and/or tissue fragments have been loaded into the organ cavities (4, 4a, 4b). Preferably the organ-on-a-chip device or its parts are packaged separately in a sterile environment. In a preferred embodiment the assembled organ on a chip device is sterilize-able by autoclaving and/or irradiation.

The medium layer (12) typically has a thickness of between 0.5 mm to 20 mm, preferably 0.5, 1.0, 1.5, 2.0, 3.0, 4.0, 5.0, 6.0, 7.0, 8.0, 9.0, 10.0, 11.0, 12.0, 13.0, 14.0, 15.0, 16.0, 17.0, 18.0, 19.0, 20.0 mm and comprises relatively simple structures including the medium feed reservoir (2) and/or the medium waste reservoir (5), which are sized to provide and/or receive a suitable amount of medium during operation. Thus, it is preferred that the medium layer is manufactured by casting from, e.g. a synthetic polymer, preferably PS, PC, PA, PI, PEEK, PPSE, EP, UP, PF, PDMS, MF, CA, PTFE and mixtures thereof, in particular PS, PC, and polysiloxane, preferably PDMS. As outlined above, it is preferred that the self-contained organ-on-a-chip device (1) of the present invention comprises one or more openings each allowing access to an organ growth section (3), preferably an organ cavity (4) and/or stem cell cavity (9). These openings are used in preferred embodiments to load the cells and/or tissue fragments into the respective organ cavity (4, 4a, 4b). Thus, the medium layer (12) preferably comprises cut outs corresponding in size and number to the organ growth sections (3) in the organ growth section layer (13).

In a preferred embodiment the medium feed reservoirs (2) and/or the medium waste reservoirs (5) are arranged in the medium layer (12). These structures are preferably disposed in the medium layer in such a way that they do not to interfere with the opening(s) that is(are) provided in a preferred embodiment in the medium layer to allow access of gaseous medium to the organ growth section (3) and organ cavities (4, 4a, 4b), respectively, which are disposed beneath the medium layer in the organ growth section layer (13). The organ growth section layer (13) preferably comprises all structures as outlined in more detail above, to provide the necessary environment including structural, chemical and physical cues, for differentiation and maintenance of tissues, organoids and organs. It, thus, will comprise the microstructure for, e.g. bone or cartilage development, for development of vascularised skin or nerve growth. Due to the small size of the structures required photolithographic techniques are often used and, accordingly, it is preferred that the materials are similar to those materials commonly used in the field of semi-conductor technology including SiO₂, GaAs, glass or combinations thereof.

In a preferred embodiment the organ growth section layer (13) comprises or consists of an upper closing layer (14), an organ cavity layer (15) and a lower closing layer (16). The three layers together delimit the organ growth section (3), wherein the upper layer delimits the upper end of the organ growth section (3) and the organ cavities (4, 4a, 4b), respectively, the organ cavity layer provides the sides of the organ cavities and the lower closing layer delimits the lower end of the organ growth section (3) and the organ cavities (4, 4a, 4b), respectively.

The upper closing layer (14) preferably has a thickness of between 20 µm to 2 mm, preferably 30, 40, 50, 60, 70, 80, 90, 100, 110, 120, 130, 140, 150, 160, 170, 180, 190, 200, 250, 300, 350, 400, 450, 500, 550, 600, 700, 800, 900, 1000, 1200, 1300, 1400, 1500, 1600, 1700, 1800, 1900 or 2000 µm. It fluidically separates the microfluidic channels in the organ growth layer from the medium layer. However, it comprises openings allowing fluid communication with corresponding openings in the medium layer to, e.g. allow the flow of medium out of and back into the medium layer (12). Further openings may be provided to allow access to the organ growth section (3), preferably to the organ cavities (4, 4a, 4b) when loading the cells into the organ cavities (4, 4a, 4b) and/or stem cell cavity (9), which may be later closed by appropriate means as outlined above. The upper closing layer (14) may further comprise organ specific surface structures in the area of an organ growth section (3), which partly or entirely cover the organ growth section (3) or organ cavity (4, 4a, 4b). Preferably the material of the upper closing layer (14) is SiO₂ or glass, most preferably glass.

The organ cavity layer (15) comprises one or more organ cavities (4, 4a, 4b) and/or stem cell cavities (9) and optionally micro-fluidic channels. When stating that the organ cavity layer comprises organ cavities it is meant that the majority of the volume of the organ cavity (4, 4a, 4b) is provided in the organ cavity layer (15), which provides the sides of the organ cavity. The organ cavity layer (15) preferably has a thickness of between 100 µm to 10 mm, preferably of 100, 110, 120, 130, 140, 150, 160, 170, 180, 190 200, 250, 300, 350, 400, 550, 600, 650, 700, 750, 800, 850, 900, 1000 µm, 1.5, 1.6, 1.7, 1.8, 1,9 or 2.0 mm. Particular preferred thicknesses are between 250 to 750 µm to allow constant monitoring of the cells in the organ cavity and/or the stem cell cavity my transmission microscopy. The thickness of the organ cavity layer (15) is chosen in such that an organ cavity (4, 4a, 4b) of the required volume is provided. Preferably the material of the organ growth layer (15) is SiO₂ or glass, most preferably SiO₂.

The lower closing layer (16) preferably has a thickness of between 20 µm to 2 mm, preferably 30, 40, 50, 60, 70, 80, 90, 100, 110, 120, 130, 140, 150, 160, 170, 180, 190 200, 250, 300, 350, 400, 550, or 600 µm. It fluidically separates the microfluidic channels and/or openings in the organ cavity layer (15) from the outside environment, i.e. preferably it does not have an opening. Preferably the material of the lower closing layer is SiO₂ or glass, most preferably glass. Preferably the lower closing layer (16) comprises one or more of the following: heating means (11), sensor means, preferably temperature sensing means, or electric connectors for connecting the device to corresponding electric connectors (19) of a holding means (18).

As outlined above the organ-on-a-chip device of the present invention may be delivered completely assembled to the point of use and may also comprise all media and/or supplements required for the growth and differentiation of the respective tissues, organoids and/or organs. Thus, in a preferred embodiment the medium reservoir (2) comprises a cell growth medium.

In a further aspect the present invention relates to a method of manufacturing a self-contained organ-on-a-chip device (1), comprising the steps of bonding a medium layer (12) fluid-tight to a growth section layer (13) or parts thereof. Such bonding may be affected by art known adhesives or depending on the respective materials by welding.

The organ-on-a-chip device may also comprise a source of energy, e.g. a battery to provide certain functions, e.g. micropumping, sensor functions, independent from any power source that may by attached to the organ-on-a-chip device through, e.g. a holding means (18) as outlined below. Additionally, the organ-on-a-chip device may comprise signalling means, e.g. LEDs, radiation transmitters to communicate the status of the organ-on-a-chip device to the outside. For example it is envisioned that a LED on the organ-on-a-chip device flashes, if the temperature leaves a pre-set temperature range.

The self contained organ-on-a-chip device may be moved around due to the fact that it preferably comprises all features for maintaining the cell, tissues, organoids, and/or organs independently. However, in a preferred embodiment the organ-on a chip device is placed into a specially adapted supply unit (17) for holding the self-contained organ-on-a-chip device (1) during operation. This supply unit (17) comprises:
(a) holding means (18) for releasably engaging the self-contained organ-on-a-chip device (1), and
(b) electric connectors (19) for connecting to corresponding connectors on the self-contained organ-on-a-chip device (1) with the supply unit (17).

The supply unit (17) typically comprises indicator means like, e.g. light indicators or sound indicators to alert an operator of the device to a change in the condition of the device like, temperature, oxygenation, pH etc. While it is envisioned to integrate the circuitry required to regulate and evaluate actuating means and sensors comprised in the organ-on-a-chip device (1), it is also possible to integrate these functions on the supply unit. This is advantageous in those embodiments, wherein the organ-on-a-chip device is a single use device, that will be thrown away after one incubation period. Accordingly, it is preferred that the supply unit (17) comprises regulating means. Typically, these will determine, e.g. the temperature within the organ-on-a-chip device, the flow of the fluids within the organ-on-a-chip device, or the electric stimulation, which may be required by certain tissues and will adjust these parameter according to, e.g. preset parameters,

In a preferred embodiment the supply unit (17) comprises a holding means (18), which allows holding at least two organ-on-a-chip devices (1) on top of each other.

In a further aspect the present invention relates to a method of establishing an organ and/or organoid in the self-contained organ-on-a-chip device (1). This method comprises the steps of:
(a) loading a suspension of cells or a tissue slice into one or more organ cavities (4, 4a, 4b), preferably into a self-contained organ-on-a-chip device (1) according to the first, second and third embodiment described above, and
(b) fluid-tight sealing of the one or more organ cavities (4, 4a, 4b).

It is possible to directly load the cells or tissue slice(s) into the organ cavity through an opening, e.g. in the upper closing layer (14) with an appropriate means like, e.g. a microsyringe or to flow cells with the medium through the organ cavity, which will then adhere to the structures and surfaces provided therein. Such loading may be manually or fully automatic. In the later case it is preferred that a stepper device, that loads any required medium, cell or substance through the opening of the organ growth section (3), which may be sealed, resealed or self-sealing as the case may be. The type of cells introduced into the organ cavities (4, 4a, 4b) will depend on the organoid or organ to establish. Preferably, the suspension of cells comprises totipotent or pluripotent stem cells, lineage committed cells, differentiated cells, extracellular matrix components or mixtures thereof. It is particularly preferred to load tissue slices into the organ cavities (4, 4a, 4b), since they merely have to reassemble within the organ cavity without a requirement for correct differentiation, which may be the case, if a organoid or organ is established from stem cells or other more differentiated progenitor cells.

To seal the organ cavity (4, 4a, 4b) from the environment after the cells have been loaded into the organ cavity sealants like fibrin glue, biocompatible polymer foil spray-on bandage, or products of coagulation may be used. Preferably the sealant will provide a fluid-tight but gaspermeable layer or membrane across the openings of the organ cavities (4, 4a, 4b).

In a subsequent step the self-contained organ-on-a-chip device (1) is incubated until a organ or organoid is formed. Typically, an incubation for at least 1, 2, 3, 4, 5, 6, 7, 8, 9, 10, 11, 12, 13 or 14 days is required until such organ or organoid formation is completed.

A hallmark of the organ-on-a-chip device is its independence from external supply, in particular of medium and/or disposal of waste, since appropriated fluidic connections and reservoir are already provided within the self-contained organ-on-a-chip device. the incubation is preferably carried out without external control of the temperature, and/or without providing a defined atmosphere and/or without providing external sterility.

In a further aspect the present invention relates to a method of testing the effect of one or more test compounds on one or more organs and/or organoids established in a self-contained organ-on-a-chip device (1). This method comprises the following steps:
(a) providing a self-contained organ-on-a-chip device (1) comprising one or more organs and/or organoids or
   carrying out the method of establishing an organ and/or organoid in a self-contained organ-on-a-chip device (1) as outlined above.
(b) adding one or more test compounds to the organ and/or organoid
(c) assessing the organ and/or organoid microscopically and/or
   determining one or more parameter determinable by one or more sensors.

The sensors used in this method may be sensors (8, 8a, 8b), which monitor the medium flowing out of the organ-on-a-chip device or may be sensors located within the organ cavity.

In a further aspect the present invention relates to the use of the self-contained organ-on-a-chip device (1) comprising one or more organs and/or organoids for testing the effects of one or more test compounds on the organs or organoids or for examining organ or organoid functions. Preferably the efficacy, side-effects, biosafety or mode of action of the one or more test compounds is determined.

### List of reference numbers

- (1): self-contained organ-on-a-chip device
- (2): medium feed reservoir,
- (3): organ growth section,
- (4, 4a, 4b): organ cavities,
- (5): medium waste reservoir,
- (6): microfluidic feed channel,
- (7, 7a, 7b): microfluidic waste channels,
- (8, 8a, 8b): sensors,
- (9): stem cell cavity,
- (9a): neonatal stem cell niche cavity
- (9b): pre/postnatal stem cell niche cavity
- (9c): adult quiescence-promoting stem cell niche cavity
- (10): outlet of the microfluidic feed channel (6),
- (11): heating means,
- (12): medium layer,
- (13): organ growth section layer,
- (14): upper closing layer,
- (15): organ cavity layer,
- (16): lower closing layer,
- (17): supply unit,
- (18): holding means,
- (19): electric connectors,
- (20): overheat indicator means,
- (21): secondary fluid flow,
- (22): impedance measuring means,
- (23): temperature sensor.

The invention is summarized as follows:
1. A self-contained organ-on-a-chip device (1) comprising:
   (a) at least one medium feed reservoir (2), and
   (b) at least one organ growth section (3) comprising at least one organ cavity (4, 4a, 4b), and
   wherein the medium feed reservoir (2) is connected to the at least one organ growth section (3) by a microfluidic feed channel (6) and the at least one organ cavity (4, 4a, 4b).
2. A self-contained organ-on-a-chip device (1) comprising
   (a) at least one organ growth section (3) comprising at least one organ cavity (4, 4a, 4b), and
   (b) wherein the at least one organ cavity (4, 4a, 4b) comprises and/or is connected to at least one sensor (8, 8a, 8b).
3. The self-contained organ-on-a-chip device (1) according to item 2, further comprising at least one medium feed reservoir (2) wherein the medium feed reservoir (2) is connected to the at least one organ growth section (3) by a microfluidic feed channel (6) and the at least one organ cavity (4, 4a, 4b).
4. The self-contained organ-on-a-chip device (1) of items 1 to 3, wherein the organ growth section (3) comprises a stem cell cavity (9).
5. A self-contained organ-on-a-chip device (1) comprising:
   (a) at least one organ growth section (3) comprising at least one organ cavity (4, 4a, 4b), and
   (b) wherein the organ growth section (3) comprises a stem cell cavity (9).
6. The self-contained organ-on-a-chip device (1) according to item 5, further comprising at least and one medium feed reservoir (2), wherein the medium feed reservoir (2) is connected to the at least one organ growth section (3) by a microfluidic feed channel (6).
7. The self-contained organ-on-a-chip device (1) according to items 1 to 6, further comprising at least one medium waste reservoir (5), wherein the at least one organ cavity (4, 4a, 4b) is connected to the at least one medium waste reservoir (5) by a microfluidic waste channel (7).
8. The self-contained organ-on-a-chip device (1) according to items 1 to 7, wherein at least one sensor (8, 8a, 8b) is arranged between the at least one organ cavity (4, 4a, 4b) and at least one medium waste reservoir (5) and/or within the at least one organ cavity.
9. The self-contained organ-on-a-chip device (1) of items 4 to 8, wherein the stem cell cavity (9) has a diameter of less than 100 µm.
10. The self-contained organ-on-a-chip device (1) of items 4 to 9, wherein the stem cell cavity is cylindrical.
11. The self-contained organ-on-a-chip device (1) of items 4 to 10, wherein the stem cell cavity is fluidly connected to the at least one organ cavity (4, 4a, 4b) by an opening of less than 80 µm.
12. The self-contained organ-on-a-chip device (1) of items 1 to 11, wherein the organ cavity (4) comprises structures selected from the group consisting of:
   (a) biodegradable micro-carriers;
   (b) pressurizing means;
   (c) a collagen web;
   (d) calcification zones;
   (e) capillaries connected to a further medium feed reservoir;
   (f) at least two reservoirs each provided with a pulsative pressurizing means for providing a secondary flow through the organ cavity;
   (g) one or more electrodes;
   (h) fibrin gels;
   (i) synthetic polypeptide gels;
   (j) cross linked proteins;
   (k) woven and/or non woven polymeric fibres;
   (l) electromagnetic field forces;
   (m) micropumps;
   (n) sensor substances;
   (o) sensors; and
   (p) optical fibres.
13. The self-contained organ-on-a-chip device (1) of items 1 to 12, wherein the growth section comprises two or more organ cavities (4, 4a, 4b), that are radially arranged with respect to an outlet (10) of the microfluidic feed channel (6).
14. The self-contained organ-on-a-chip device (1) of item 13, wherein the organ cavities (4, 4a, 4b) are in the form of conical segments of a disc.
15. The self-contained organ-on-a-chip device (1) of items 13 or 14, wherein the stem cell cavity (9) is arranged opposite the outlet (10) of the microfluidic feed channel (6).
16. The self-contained organ-on-a-chip device (1) of items 1 to 15, wherein the one or more organ cavities (4, 4a, 4b) and/or the at least one sensor (8, 8a, 8b) are microscopable.
17. The self-contained organ-on-a-chip device (1) of items 2 to 16, wherein the stem cell cavity (9) is microscopable.
18. The self-contained organ-on-a-chip device (1) of items 1 to 17, wherein the sensor (8, 8a, 8b) is selected from the group consisting of:
   (a) pH sensor;
   (b) pO₂ sensor;
   (c) analyte capture sensor;
   (d) conductivity sensor;
   (e) plasmon resonance sensor;
   (f) temperature sensor;
   (g) CO₂ sensor;
   (h) NO sensor;
   (i) chemotaxis sensor;
   (j) cytokine sensor;
   (k) ion sensor;
   (l) potentiometric sensor;
   (m) amperometric sensor;
   (n) flow-through-sensor;
   (o) fill sensor;
   (p) impedance sensor;
   (q) conductivity sensor;
   (r) electromagnetic field sensor;
   (s) surface acoustic wave (SAW) sensor; and
   (t) metabolic sensor
19. The self-contained organ-on-a-chip device (1) of items 1 to 18, wherein two or more sensors (8, 8a, 8b) are arranged in the flow path from one organ cavity (4) to the medium waste reservoir (5).
20. The self-contained organ-on-a-chip device (1) of items 1 to 19, wherein the medium waste reservoir (5) comprises a hydrophilic material and/or a micro pump is arranged in the flow path between the medium feed reservoir (2) and the medium waste reservoir (5).
21. The self-contained organ-on-a-chip device (1) of items 1 to 20, further comprising heating means, arranged to heat the at least one medium feed reservoir (2) and/or the at least one organ cavity (4, 4a, 4b).
22. The self-contained organ-on-a-chip device (1) of item 21, further comprising heating means (11), arranged to heat the at least one medium feed reservoir (2) and/or the at least one organ cavity (4, 4a, 4b).
23. The self-contained organ-on-a-chip device (1) of item 22, wherein the heating means (11) consist of indium tin oxide, platinum and gold.
24. The self-contained organ-on-a-chip device (1) of items 1 to 23, further comprising a temperature sensor arranged to determine the temperature in the at least one medium feed reservoir (2) and/or the at least one organ cavity (4, 4a, 4b).
25. The self-contained organ-on-a-chip device (1) of items 1 to 24, wherein the device comprises at least a medium layer (12) and an organ growth section layer (13).
26. The self-contained organ-on-a-chip device (1) of item 25, wherein the medium feed reservoirs (2) and/or the medium waste reservoirs (5) are arranged in the medium layer (12).
27. The self-contained organ-on-a-chip device (1) of items 25 or 26, wherein the medium layer (12) comprises one or more openings each allowing access to an organ growth section (3), preferably an organ cavity (4) and/or stem cell cavity (9).
28. The self-contained organ-on-a-chip device (1) of items 25 to 27, wherein the organ growth section layer comprises an upper closing layer (14), an organ cavity layer (15) and a lower closing layer (16).
29. The self-contained organ-on-a-chip device (1) of item 28, wherein the upper closing layer (14) comprises one or more openings each allowing access to an organ growth section (3), preferably an organ cavity (4) and/or stem cell cavity (9) and optionally organ specific surface structures in the area of an organ growth section (3).
30. The self-contained organ-on-a-chip device (1) of item 29, wherein the organ cavity layer (15) comprises one or more organ cavities (4, 4a, 4b) and/or stem cell cavities (9) and optionally micro-fluidic channels.
31. The self-contained organ-on-a-chip device (1) of item 30, wherein the lower closing layer (16) comprises one or more of the following: heating means (11), temperature sensing means (23), or electric connectors for connecting the device to corresponding electric connectors (19) of a holding means (18).
32. The self-contained organ-on-a-chip device (1) of items 1 to 31, wherein the medium reservoir (2) comprises a cell growth medium.
33. A method of manufacturing a self-contained organ-on-a-chip device (1) of items 1 to 32, comprising the steps of bonding a medium layer (12) fluid-tight to a growth section layer (13) or parts thereof.
34. A supply unit (17) for holding the self-contained organ-on-a-chip device (1) of items 1 to 33 during operation comprising:
   (a) holding means (18) for releasably engaging the self-contained organ-on-a-chip device (1), and
   (b) electric connectors (19) for connecting to corresponding connectors on the self-contained organ-on-a-chip device (1) to the supply unit (17).
35. The supply unit (17) of item 34, further comprising indicator means.
36. The supply unit (17) of item 34 or 35, further comprising regulating means.
37. The supply unit (17) of items 34 to 36, configured to hold at least two organ-on-a-chip devices (1) on top of each other.
38. A method of establishing an organ and/or organoid in a self-contained organ-on-a-chip device (1) of items 1 to 32, comprising the steps of:
   (a) loading a suspension of cells or a tissue slice into one or more organ cavities (4, 4a, 4b), and
   (b) fluid-tight sealing of the one or more organ cavities (4, 4a, 4b).
39. The method of item 38, wherein the suspension of cells comprises totipotent or pluripotent stem cells, lineage committed cells, differentiated cells, extracellular matrix components or mixtures thereof.
40. The method of item 38 or 39, wherein fibrin glue, biocompatible polymer foil spray-on bandage, or products of coagulation is used in step (b).
41. The method of items 38 to 40, comprising the further step of incubating the self-contained organ-on-a-chip device (1) until an organ or organoid is formed.
42. The method of item 41, wherein the incubation is carried out without external control of the temperature, and/or without providing a defined atmosphere and/or without providing external sterility.
43. A method of testing the effect of one or more test compounds on one or more organs and/or organoids established in a self-contained organ-on-a-chip device (1) of items 1 to 32, comprising:
   (a) providing a self-contained organ-on-a-chip device (1) of items 1 to 32 comprising one or more organs and/or organoids or
      carrying out the method of establishing an organ and/or organoid in a self-contained organ-on-a-chip device (1) of items 38 to 42,
   (b) adding one or more test compounds to the organ and/or organoid
   (c) assessing the organ and/or organoid microscopically; and/or
      determining one or more parameter determinable by one or more sensors (8, 8a, 8b).
44. Use of the self-contained organ-on-a-chip device (1) of items 1 to 32 comprising one or more organs and/or organoids for testing the effects of one or more test compounds on the organs or organoids or for examining organ or organoid functions.
45. Use of item 44, wherein the efficacy, side-effects, biosafety or mode of action of the one or more test compounds is determined.

## Claims

1. A self-contained organ-on-a-chip device (1) comprising
(a) at least one organ growth section (3) comprising at least one organ cavity (4, 4a, 4b), and
(b) wherein the at least one organ cavity (4, 4a, 4b) comprises and/or is connected to at least one sensor (8, 8a, 8b)
wherein the organ cavity is designed to support the formation of liver segments by providing a spaced environment, which allows growth of liver segments with a maximal length of 500 µm, by providing an O₂ gradient across the entire liver segment and providing to the polar hepatocytes a blood side and a gall side.

2. The self-contained organ-on-a-chip device (1) according to claim 1, further comprising at least one medium feed reservoir (2) wherein the medium feed reservoir (2) is connected to the at least one organ growth section (3) by a microfluidic feed channel (6) and the at least one organ cavity (4, 4a, 4b).

3. A self-contained organ-on-a-chip device (1) comprising:
(a) at least one medium feed reservoir (2), and
(b) at least one organ growth section (3) comprising at least one organ cavity (4, 4a, 4b), and
wherein the medium feed reservoir (2) is connected to the at least one organ growth section (3) by a microfluidic feed channel (6) and the at least one organ cavity (4, 4a, 4b) and wherein the organ cavity is designed to support the formation of liver segments by providing a spaced environment, which allows growth of liver segments with a maximal length of 500 µm, by providing an O₂ gradient across the entire liver segment and providing to the polar hepatocytes a blood side and a gall side.

4. The self-contained organ-on-a-chip device (1) of claims 1 to 3, wherein the organ growth section (3) comprises a stem cell cavity (9).

5. A self-contained organ-on-a-chip device (1) comprising:
(a) at least one organ growth section (3) comprising at least one organ cavity (4, 4a, 4b), and
(b) wherein the organ growth section (3) comprises a stem cell cavity (9)
wherein the organ cavity is designed to support the formation of liver segments by providing a spaced environment, which allows growth of liver segments with a maximal length of 500 µm, by providing an O₂ gradient across the entire liver segment and providing to the polar hepatocytes a blood side and a gall side.

6. The self-contained organ-on-a-chip device (1) according to claim 5, further comprising at least and one medium feed reservoir (2), wherein the medium feed reservoir (2) is connected to the at least one organ growth section (3) by a microfluidic feed channel (6).

7. The self-contained organ-on-a-chip device (1) according to claims 1 to 6, further comprising at least one medium waste reservoir (5), wherein the at least one organ cavity (4, 4a, 4b) is connected to the at least one medium waste reservoir (5) by a microfluidic waste channel (7).

8. The self-contained organ-on-a-chip device (1) according to claims 1 to 7, wherein at least one sensor (8, 8a, 8b) is arranged between the at least one organ cavity (4, 4a, 4b) and at least one medium waste reservoir (5) and/or within the at least one organ cavity.

9. The self-contained organ-on-a-chip device (1) of claims 1 to 8, wherein the growth section comprises two or more organ cavities (4, 4a, 4b), that are radially arranged with respect to an outlet (10) of the microfluidic feed channel (6) wherein preferably the organ cavities (4, 4a, 4b) are in the form of conical segments of a disc.

10. The self-contained organ-on-a-chip device (1) of claims 1 to 9, wherein the sensor (8, 8a, 8b) is selected from the group consisting of:
(a) pH sensor;
(b) pO₂ sensor;
(c) analyte capture sensor;
(d) conductivity sensor;
(e) plasmon resonance sensor;
(f) temperature sensor;
(g) CO₂ sensor;
(h) NO sensor;
(i) chemotaxis sensor;
(j) cytokine sensor;
(k) ion sensor;
(l) potentiometric sensor;
(m) amperometric sensor;
(n) flow-through-sensor;
(o) fill sensor;
(p) impedance sensor;
(q) conductivity sensor;
(r) electromagnetic field sensor;
(s) surface acoustic wave (SAW) sensor; and
(t) metabolic sensor

11. A method of manufacturing a self-contained organ-on-a-chip device (1) of claims 1 to 10, comprising the steps of bonding a medium layer (12) fluid-tight to a growth section layer (13) or parts thereof.

12. A supply unit (17) for holding the self-contained organ-on-a-chip device (1) of claims 1 to 10 during operation comprising:
(a) holding means (18) for releasably engaging the self-contained organ-on-a-chip device (1), and
(b) electric connectors (19) for connecting to corresponding connectors on the self-contained organ-on-a-chip device (1) to the supply unit (17).

13. A method of establishing an organ and/or organoid in a self-contained organ-on-a-chip device (1) of claims 1 to 10, comprising the steps of:
(a) loading a suspension of cells or a tissue slice into one or more organ cavities (4, 4a, 4b), and
(b) fluid-tight sealing of the one or more organ cavities (4, 4a, 4b).

14. A method of testing the effect of one or more test compounds on one or more organs and/or organoids established in a self-contained organ-on-a-chip device (1) of claims 1 to 10, comprising:
(a) providing a self-contained organ-on-a-chip device (1) of claims 1 to 10 comprising one or more organs and/or organoids or
carrying out the method of establishing an organ and/or organoid in a self-contained organ-on-a-chip device (1) of claim 13,
(b) adding one or more test compounds to the organ and/or organoid
(c) assessing the organ and/or organoid microscopically; and/or
determining one or more parameter determinable by one or more sensors (8, 8a, 8b).

15. Use of the self-contained organ-on-a-chip device (1) of claims 1 to 10 comprising one or more organs and/or organoids for testing the effects of one or more test compounds on the organs or organoids or for examining organ or organoid functions.
